(19) Europäisches Patentamt
European Patent Office
Office européen des brevets

(11) EP 1 845 157 A1

(12) EUROPEAN PATENT APPLICATION
published in accordance with Art. 158(3) EPC

(43) Date of publication:
17.10.2007 Bulletin 2007/42

(21) Application number: 06711712.7

(22) Date of filing: 16.01.2006

(51) Int Cl.:
*C12N 15/09* (2006.01) *C12M 1/00* (2006.01)
*C12Q 1/68* (2006.01) *G01N 33/53* (2006.01)
*G01N 37/00* (2006.01)

(86) International application number:
PCT/JP2006/300431

(87) International publication number:
WO 2006/075735 (20.07.2006 Gazette 2006/29)

(84) Designated Contracting States:
DE FR GB

(30) Priority: 14.01.2005 US 643603 P
11.03.2005 US 660334 P

(71) Applicant: NGK INSULATORS, LTD.
Nagoya-city, Aichi 467-8530 (JP)

(72) Inventors:
- YOSHIDA, Yasuko,
c/o NGK Insulators, Ltd.
Nagoya-city, Aichi, 4678530 (JP)
- YAMADA, Kazunari,
c/o NGK Insulators, Ltd.
Nagoya-city, Aichi, 4678530 (JP)
- NIWA, Kousuke,
c/o NGK Insulators, Ltd.
Nagoya-city, Aichi, 4678530 (JP)

(74) Representative: Paget, Hugh Charles Edward
Mewburn Ellis LLP
York House
23 Kingsway
London WC2B 6HP (GB)

# (54) ARRAY AND HYBRIDIZATION METHOD

(57) The present invention provides a hybridization method suited for using a signal probe. An array of the present invention comprises: a substrate; a nucleic acid probe that is fixed to the substrate and is hybridized with a sample to have a signal change; and at least one cavity that is filled with a specific liquid containing the sample and causing the hybridization of the nucleic acid probe with the sample. The array in this arrangement effectively enhances the reproducibility and the efficiency of hybridization with the signal probe.

EP 1 845 157 A1

SEQUENCE LISTING

<110> NGK Insulators, Ltd.

<120> Array and Hibridization Method

<130> FNNGA012WO

<150> US60/643603
<151> 2005-01-14
<150> US60/660334
<151> 2005-03-11

<160> 17

<170> PatentIn version 3.3

<210> 1
<211> 20
<212> DNA
<213> Artificial

<220>
<223> synthetic origonucleotide

<220>
<221> misc_feature
<222> (10)..(10)
<223> uracil derivative modified with 1-pyrenyl

<400> 1
ttttcacttu agtgtatgcc 20

<210> 2
<211> 20
<212> DNA
<213> Artificial

<220>
<223> synthetic origonucleotide

<400> 2
ttttcacttt agtgtatgcc 20

<210> 3

<211> 20
<212> DNA
<213> Artificial

<220>
<223> synthetic origonucleotide

<220>
<221> misc_feature
<222> (10)..(10)
<223> uracil derivative modified with 1-pyrenyl

<220>
<221> misc_feature
<222> (15)..(15)
<223> uracil derivative modified with 1-pyrenyl

<400> 3
ttttcacttu agtguatgcc 20

<210> 4
<211> 20
<212> DNA
<213> Artificial

<220>
<223> synthetic origonucleotide

<220>
<221> misc_feature
<222> (11)..(11)
<223> uracil derivative modified with 1-pyrenyl

<400> 4
tgaagggctt utgaactctg 20

<210> 5
<211> 20
<212> DNA
<213> Artificial

<220>
<223> synthetic origonucleotide

<220>
<221> misc_feature
<222> (11)..(11)
<223> uracil derivative modified with 1-pyrenyl

<400> 5
cagtacagat utgaactctg 20

<210> 6
<211> 20
<212> DNA
<213> Artificial

<220>
<223> synthetic origonucleotide

<220>
<221> misc_feature
<222> (10)..(10)
<223> cytosin derivative modified with 1-pyrenyl

<400> 6
ttttcacttc agtgtatgcc 20

<210> 7
<211> 20
<212> DNA
<213> Artificial

<220>
<223> synthetic origonucleotide

<400> 7
ttttcacttc agtgtatgcc 20

<210> 8
<211> 20
<212> DNA
<213> Homo sapiens

<400> 8
ggcatacact aaagtgaaaa 20

<210> 9
<211> 20
<212> DNA
<213> Homo sapiens

<400> 9
ggcatacact gaagtgaaaa 20

<210> 10
<211> 189
<212> DNA
<213> Homo sapiens

<400> 10
tatgatgtgt ttggagccca gtcacccttt ggtggctaca agatgtcggg gagtggccgg 60

gagttgggcg agtacgggct gcaggcatac actaaagtga aaactgtcac agtcaaagtg 120

cctcagaaga actcataaga atcatgcaag cttcctccct cagccattga tggaaagttc 180

agcaagatc 189

<210> 11
<211> 189
<212> DNA
<213> Homo sapiens

<400> 11
tatgatgtgt ttggagccca gtcacccttt ggtggctaca agatgtcggg gagtggccgg 60

gagttgggcg agtacgggct gcaggcatac actgaagtga aaactgtcac agtcaaagtg 120

cctcagaaga actcataaga atcatgcaag cttcctccct cagccattga tggaaagttc 180

agcaagatc 189

<210> 12
<211> 129
<212> DNA
<213> Homo sapiens

<400> 12
atgatgagtc tccggggctc tatgggtttc tgaatgtcat cgtccactca gccactggat 60

ttaagcagag ttcaaatctg tactgcaccc tggaggtgga ttcctttggg tattttgtga 120

ataaagcaa 129

<210> 13
<211> 129
<212> DNA
<213> Homo sapiens

<400> 13
atgatgagtc tccggggctc tatgggtttc tgaatgtcat cgtccactca gccactggat 60

ttaagcagag ttcaaaagcc cttcagcggc cagtagcatc tgactttgag cctcagggtc 120

tgagtgaag 129

<210> 14
<211> 18
<212> DNA
<213> Artificial

<220>
<223> synthetic origonucleotide

<400> 14
tatgatgtgt ttggagcc 18

<210> 15
<211> 18
<212> DNA
<213> Artificial

<220>
<223> synthetic origonucleotide

<400> 15
gatcttgctg aactttcc 18

<210> 16
<211> 18
<212> DNA
<213> Artificial

<220>

<223> synthetic origonucleotide

<400> 16
tatgatgtgt ttggagcc 18

<210> 17
<211> 18
<212> DNA
<213> Artificial

<220>
<223> synthetic origonucleotide

<400> 17
gatcttgctg aactttcc 18

## Description

### Technical Field

The present invention relates to an array carrying a probe that is hybridized with a specific base to have a detectable signal change, as well as to an improved structure of the probe and applications of the array and the probe.

### Background Art

cDNA probes and oligonucleotide probes are typically fixed on the surface of glass substrates to form DNA microarrays and are used for gene analysis of the humans and other organisms. The human gene analysis measures various gene-related signals of, for example, the gene polymorphisms, the gene mutations, and the gene expressions for the purpose of genetic diagnosis.

Hybridization of the probes with DNA samples is utilized for the measurement of the gene-related signals. A DNA sample labeled with, for example, a fluorescent substance is supplied to a probe fixed to a solid phase support and is hybridized. After wash-out of the remaining unhybridized DNA sample, the labeling quantity (for example, fluorescence signal intensity) derived from the labeling substance of the DNA sample hybridized with the probe is measured as the gene-related signal of, for example, the polymorphism. The labeling step, the hybridization step, and the washing step accordingly have variation factors of the final labeling quantity.

The amount of the labeled DNA sample supplied to the probe may not be constant but may be varied. This causes a significant variation in labeling quantity of the resulting hybridization product. The hybridization is affected by diverse factors and accordingly has difficulty in performance of the high reproducibility. There is also the potential for mishybridization of the probe with the DNA sample. The hybridization step accordingly has a variation factor of the labeling quantity. Insufficient washing makes the residue of the labeled DNA sample and thus causes noise and a variation factor of the labeling quantity. The conventional gene analysis uses a control for signal correction and devises the design, the arrangement, and the analysis of probes to eliminate the potential effects of mishybridization. Various measures have also been proposed to stabilize the hybridization step.

The electrochemical technique with intercalators has been proposed as disclosed in Japanese Patent Laid-Open Gazette No. 2004-357570. Base-discriminating fluorescent (BDF) nucleobases have been developed to have fluorescence by hybridization with specific bases as disclosed in Japanese Patent Laid-Open Gazette No. 2004-168672 and No. 2004-166522. A probe containing a base-discriminating fluorescent nucleobase spontaneously has a specific signal change by pairing with a specific base included in a target sequence as the detection object.

### Disclosure of the Invention

The use of the signal probe having a signal change by hybridization with a specific base does not require the labeling operation of each DNA sample. The use of the signal probe is accordingly expected to eliminate or at least reduce the error caused by the labeling step. The improved specificity of the signal probe is also expected to eliminate or at least reduce the error caused by mishybridization. The conventional method requires multiple different types of probes for reduction of the mishybridization-inducing measurement errors. The use of the signal probe also does not require the manufacture and the arrangement of these multiple different types of probes. The stable and high hybridization efficiency is, however, essential to attain such expected advantages of the signal probe. An effective correction technique is also required to adequately correct a variation in signal intensity due to the varying supply amount of the sample to the DNA microarray for each measurement or each gene.

The present invention thus aims to provide a technique that is adequate for gene analysis using such a signal probe or a probe with a hybridization-induced signal change. More specifically the invention aims to provide a hybridization method that is suitable for the signal probe. The invention also aims to provide an improved structure of the signal probe and a solid phase support with fixation of the signal probe.

The inventors of the present invention have made studies and examinations and found that hybridization in a specific state effectively enhances the reproducibility and the efficiency of hybridization with the signal probe. The inventors have also found that a new design of the signal probe enables correction of a signal change simultaneously with detection of the signal change. Based on such findings, the inventors have completed the present invention having the structure and the arrangement described below.

The present invention provides an array, comprising: a substrate; a nucleic acid probe that is fixed to the substrate and is hybridized with a sample to have a signal change; and at least one cavity that is filled with a specific liquid containing the sample and causing the hybridization of the nucleic acid probe with the sample.

It is preferable that the array of the invention further has a cover member. In the array of the invention, the at least one cavity is preferably provided in a recess that is formed in the substrate and includes a fixation area of the

nucleic acid probe on the substrate. It is more preferable that the bottom of the recess is the fixation area of the nucleic acid probe. The at least one cavity is preferably provided in a hollow space of a cover member that is located at a certain height to face and cover over a fixation area of the nucleic acid probe on the substrate. It is more preferable that the cavity is a space that is defined by the hollow space of the cover member and the fixation area. The cover member may form part of a vessel that holds the array therein. The cover member is preferably detachably attached to the substrate. The cover member may be fastened to the array via an adhesive or a binding agent. The cover member preferably has transparency to allow detection of the signal change caused by the hybridization of the nucleic acid probe with the sample. The cover member may have an inlet to fill the at least one cavity with the specific liquid for causing the hybridization. In the cover member, an opposed area of the cover member facing a fixation area of the nucleic acid probe on the substrate may have a thickness of not less than 300 $\mu$m. The opposed area of the cover member facing the fixation area of the nucleic acid probe on the substrate may be made of one or multiple materials selected among the group consisting of glasses, polycarbonates, polyolefins, polyamides, polyimides, acrylic resins, fluorides of the acrylic resins, and polyvinyl halides.

The array of the invention may have a hydrophobic region in at least part of an exposed area exposed to inside of the cavity. At least part of the cover member may have a hydrophobic region, and the hydrophobic region may be provided in an opposed area facing a fixation area of the nucleic acid probe on the substrate. The hydrophobic region preferably has a water contact angle of not less than 30 degrees, more preferably not less than 60 degrees, still more preferably not less than 70 degrees.

It is preferable that the cavity has at least one of a height with a coefficient of variation of not higher than 50% and an average height of not less than 15 $\mu$m, where the height represents a distance between a fixation area of the nucleic acid probe on the substrate and an opposed area facing the fixation area.

It is preferable that the nucleic acid probe is hybridizable to have a fluorescence signal change that is any one or any combination of a shift in fluorescence wavelength, an increase in fluorescence intensity, and a decrease in fluorescence intensity. The nucleic acid probe preferably includes a base-discriminating fluorescent nucleobase. It is preferable that the sample is an unlabelled nucleic acid. It is preferable that the nucleic acid probe is in a range of 20 mer to 100 mer, and a detection accuracy of the signal change has a of not higher than 10% or preferably not higher than 5%.

The array of the invention may be used in at least one or multiple applications among constitution identification like single nucleotide polymorphisms, affected gene diagnosis as various gene mutations including gene chimeras, prognostic expectation, drug responsiveness detection, and drug resistance detection.

The present invention provides a hybridization method of a nucleic acid, comprising a hybridization step of filling the at least one cavity of the array of the invention with any of arrangements described above with the specific liquid containing the sample to cause the hybridization of the nucleic acid probe with the sample.

The hybridization step may cause the hybridization of the nucleic acid probe with the sample while the array is kept stationary or while the specific liquid in the at least one cavity of the array is forcibly stirred in a temporary, intermittent, or continuous manner. The sample is preferably an unlabelled nucleic acid.

The hybridization method further may further include a detection step that follows the hybridization step and detects a signal change of the nucleic acid probe induced by the hybridization. The detection step may detect the signal change, while the specific liquid containing the sample is kept in the at least one cavity after the hybridization step. The detection step may detect the signal change after the hybridization step without any washing step.

The present invention also provides a probe carrier for hybridization of a nucleic acid, comprising: a solid phase support; and a probe that is fixed to the solid phase support in an identifiable manner and is hybridized with a sample to have a signal change.

The solid phase support may be a flat plate or particles. The solid phase support may have liquid permeability or porosity. For example, the solid phase support may allow fixation of only one single type of the probe and have probe identification information selected among color, fluorescence, mark, figure, letter, character, and pattern.

It is preferable that the probe is hybridizable to have a fluorescence signal change that is any one or any combination of a shift in fluorescence wavelength, an increase in fluorescence intensity, and a decrease in fluorescence intensity. The probe preferably includes a base-discriminating fluorescent nucleobase. The sample may be an unlabelled nucleic acid. The probe carrier of the invention may be used in a state that the probe carrier is soaked in or suspended in a specific liquid containing the sample.

The present invention also provides a hybridization method of a nucleic acid, comprising a hybridization step of filling a cavity, which includes the probe carrier of the invention wit any of arrangements descrived above, with a specific liquid containing the sample to cause the hybridization of the probe with the sample.

The hybridization method may further include a detection step that follows the hybridization step and detects the signal change in the presence of the specific liquid containing the sample.

The present invention also provides a probe for hybridization of a nucleic acid, the probe comprising: (a) a characteristic detectable region where a characteristic on a base sequence of a sample is detectable as an object of

detection; and (b) a stationary detectable region where a stationary sequence of the sample, which is located in proximity to the characteristic on the base sequence detected in the characteristic detectable region (a), is detectable, wherein the stationary detectable region (b) represents any one of (1) an area with no detection of mutation, (2) an area estimated to have no mutation or have a high potential for no mutation, and (3) an area confirmed to have no mutation or have a high potential for no mutation, the probe having individually different signal changes induced by hybridization in the characteristic detectable region (a) and by hybridization in the stationary detectable region (b).

It is preferable that each of the individually different signal changes induced by the hybridization in the characteristic detectable region (a) and by the hybridization in the stationary detectable region (b) is a fluorescence signal change that is any one or any combination of a shift in fluorescence wavelength, an increase in fluorescence intensity, and a decrease in fluorescence intensity. The probe preferably includes a base-discriminating fluorescent nucleobase. The sample is preferably an unlabelled nucleic acid.

The present invention further provides a probe carrier for hybridization of a nucleic acid, the probe carrier comprising: a solid phase support; and the probe of the invention with any of the arrangements described above that is fixed to the solid phase support.

The solid phase support may be a flat plate or particles. The solid phase support may have liquid permeability or porosity. For example, the solid phase support may allow fixation of only one single type of the probe and have probe identification information selected among color, fluorescence, mark, figure, letter, character, and pattern. The probe carrier of the invention may be used in a state that the probe carrier is soaked in or suspended in a specific liquid containing the sample.

The probe may be in a range of 20 mer to 100 mer. The detection accuracy of the signal change preferably have a coefficient of variation or CV of not higher than 10%.

The probe carrier may be used at least one or multiple applications among constitution identification like single nucleotide polymorphisms, affected gene diagnosis as various gene mutations including gene chimeras prognostic expectation, drug responsiveness detection, and drug resistance detection.

The present invention further provides a nucleic acid hybridization device, comprising: the probe carrier of the invention having (a) the characteristic detectable region and (b) the stationary detectable region; and at least one cavity that is filled with a specific liquid for causing hybridization of the probe included in the probe carrier.

The nucleic acid hybridization device may have a hydrophobic region in at least part of an exposed area exposed to inside of the cavity. The hydrophobic region preferably has a water contact angle of not less than 30 degrees, more preferably not less than 60 degrees, still more preferably not less than 70 degrees. It is preferable that the hydrophobic region is made of one or multiple materials selected among the group consisting of glasses, polycarbonates, polyolefins, polyamides, polyimides, acrylic resins, fluorides of the acrylic resins, and polyvinyl halides. It is preferable that the nucleic acid hybridization device further has a cover member that covers over an opening of the at least one cavity. The solid phase support is preferably a substrate, and the cover member is preferably detachably attached to the substrate. The cover member preferably has transparency to allow detection of the signal change caused by the hybridization of the probe with the sample, and an opposed area of the cover member facing a fixation area of the probe on the solid phase support preferably has a thickness of not less than 300 μm.

In the nucleic acid hybridization device, at least part of the cover member may have a hydrophobic region. The hydrophobic region is preferably provided in an opposed area facing a fixation area of the probe on the solid phase support. It is preferable that the cavity has at least one of a height with a coefficient of variation of not higher than 50% and an average height of not less than 15 μm, where the height represents a distance between a fixation area of the probe on the solid phase support and an opposed area facing the fixation area.

The present invention further provides a nucleic acid hybridization method, comprising a hybridization step of filling a cavity, which includes the probe carrier according to any of claims 42 to 49, with a specific liquid containing the sample to cause the hybridization of the probe with the sample.

It is preferable that the hybridization step causes the hybridization of the probe while the nucleic acid hybridization device is kept stationary or while the specific liquid in the cavity is forcibly stirred in a temporary, intermittent, or continuous manner. The nucleic acid hybridization method may further has a detection step that follows the hybridization step and detects the signal change induced by the hybridization of the probe. The detection step preferably detects the signal change in the presence of the specific liquid containing the sample after the hybridization step. The detection signal may detect the signal change after the hybridization step without any washing step.

The present invention still further provides a probe for hybridization of a nucleic acid, having a stationary detectable region where a stationary sequence is detectable with regard to a subject individual or with regard to a group including a subject individual, such as a family group, a racial group, or an ethnic group, wherein the stationary detectable region represents any one of (1) an area with no detection of mutation, (2) an area estimated to have no mutation or have a high potential for no mutation, and (3) an area confirmed to have no mutation or have a high potential for no mutation, the probe having a signal change induced by hybridization in the stationary detectable region.

The signal change induced by the hybridization in the stationary detectable region of the stationary sequence

is preferably a fluorescence signal change that is any one or any combination of a shift in fluorescence wavelength, an increase in fluorescence intensity, and a decrease in fluorescence intensity. The probe preferably includes a base-discriminating fluorescent nucleobase.

Brief Description of the Drawings

Fig. 1 shows the structure of a probe suitable for detection of a polymorphism site and genotypes in ALDH2 gene;
Fig. 2 shows detection of polymorphisms with the probe of Fig. 1;
Fig. 3 shows the structure of a hybridization device;
Fig. 4 is a plan view and a sectional view showing the hybridization device of Fig. 3;
Fig. 5 shows measurement sites for space height;
Fig. 6 shows the structure of a hybridization device manufactured in Example 4;
Fig. 7 shows evaluation of hybridization with the hybridization device manufactured in Example 4;
Fig. 8 is a graph showing a variation in fluorescence signal among multiple spots of each identical base-discriminating fluorescence nucleobase probe;
Fig. 9 is a graph showing a variation in fluorescence signal among multiple spots of each identical conventional probe;
Fig. 10 is a graph showing the stirring effect of a hybridization liquid in a cavity of the hybridization device;
Fig. 11 is a graph showing the relation between the fluorescence signal derived from a base-discriminating fluorescent nucleobase for detection of a mutation site and the fluorescence signal derived from a base-discriminating fluorescent nucleobase for detection of an internal standard with a probe including these nucleobases;
Fig. 12 is a graph showing the hybridization results of non-labeled samples with probes having base-discriminating fluorescent nucleobases for detection of a chimera (breakpoint) of the bcr/abl gene and for detection of b3 in the normal bcr gene;
Fig. 13 is a graph showing the hybridization results of labeled samples with the probes having the base-discriminating fluorescent nucleobases for detection of the chimera (breakpoint) of the bcr/abl gene and for detection of b3 in the normal bcr gene;
Fig. 14 shows evaluation of a probe with fixation of a base-discriminating fluorescent nucleobase for detection of a mutation site on a solid phase support of porous particles; and
Fig. 15 shows evaluation of a probe with fixation of a base-discriminating fluorescent nucleobase for detection of a mutation site on a solid phase support of liquid-permeable flat plate.

Best Modes of Carrying Out the Invention

The array of the invention includes a substrate, a nucleic acid probe that is fixed to the substrate and is hybridized with a sample to have a signal change, and at least one cavity that is filled with a specific liquid containing the sample and causing the hybridization of the nucleic acid probe with the sample. In the array of this structure, the hybridization of the nucleic acid probe with the sample proceeds in the cavity. The presence of the cavity accelerates the hybridization and enhances the amount of the signal change of the nucleic acid probe, while reducing the variation in amount of the signal change. The nucleic acid probe of this array has a signal change induced only by the hybridization. Namely there is no signal change in the event of mishybridization. The array of the invention can thus reduce the variation in amount of the signal change induced by the hybridization and accordingly ensure gene analyses of high detection sensitivity and high accuracy. The nucleic acid probe is hybridized with the sample to have a signal change. The amount of the signal change is detectable in the presence of the specific liquid, which has been fed to the array and contains the sample. The array of the invention has the at least one cavity that is filled with the specific liquid. The specific liquid is readily kept in this cavity. The signal change is accordingly detectable without any state change after the hybridization of the nucleic acid probe with the sample. This arrangement of the invention effectively eliminates or at least reduces a potential variation in amount of the signal change, which may be caused by, for example, washing the array after the hybridization. The array of the invention enables the efficient hybridization over the whole fixation area of the nucleic acid probe on the substrate, thus enhancing the reproducibility of the hybridization.
The probe carrier of the invention is applied for hybridization of a nucleic acid. The probe carrier includes: a solid phase support; and a probe that is fixed to the solid phase support in an identifiable manner and is hybridized with a sample to have a signal change. In the probe carrier of the invention, the probe has a signal change induced only by the hybridization with the sample. Namely there is no signal change in the event of mishybridization. The probe carrier of the invention can thus stably detect a specific sequence included in the sample without strictly controlling hybridization conditions. The nucleic acid probe is hybridized with the sample to have a signal change. The amount of the signal change is detectable in the presence of the specific liquid, which has been fed to the array and contains the sample.

The probe of the invention is applied for hybridization of a nucleic acid. The probe includes: (a) a characteristic detectable region where a characteristic on a base sequence of a sample is detectable as an object of detection; and (b) a stationary detectable region where a stationary sequence of the sample, which is located in proximity to the characteristic on the base sequence detected in the characteristic detectable region(a), is detectable. The stationary detectable region (b) represents any one of (1) an area with no detection of mutation, (2) an area estimated to have no mutation or have a high potential for no mutation, and (3) an area confirmed to have no mutation or have a high potential for no mutation. The probe has individually different signal changes induced by hybridization in the characteristic detectable region (a) and by hybridization in the stationary detectable region (b).

In the probe of the invention, there is a signal change induced by hybridization with the sample in the characteristic detectable region (a) of the probe, simultaneously with a signal change induced by hybridization with the sample in the stationary detectable region (b) of the probe. The signal change observed in the stationary detectable region (b) is usable for signal correction. These two signal changes are observed on one single probe. The arrangement of the invention does not require any additional probe or any specific control for the signal correction. The probe of the invention facilitates relative quantitation and absolute determination of the polymorphisms, the mutation, and the chimera.

Best modes of carrying out the invention are described below as the applications of the hybridization method, the probe carrier, and the probe with reference to the accompanied drawings.

(Nucleic Acid Probe Having Signal Change by Hybridization)

The nucleic acid probe of the invention has a signal change induced by hybridization. The 'nucleic acid' of the invention is demanded to be at least partly hybridizable with another nucleic acid by nucleic acid base pairing. The terminology 'nucleic acid' in the specification hereof accordingly includes natural and synthetic oligonucleotides and polynucleotides, genome DNA, cDNAs, and other diverse DNAs, PCR products, mRNAs and other diverse RNAs, and peptide nucleic acids. The terminology 'hybridization' in the specification hereof represents a binding reaction of complementary chains by base pairing between nucleic acid molecules.

The 'signal change' is not specifically restricted but may be a change in any of an electrochemical signal, a color signal, a light signal, and a radiated signal. The 'probe having a signal change' may be, for example, a probe equipped with a redox unit that adopts an electrochemical approach to detect a mismatch as disclosed in Japanese Patent Laid-Open Gazette No. 2004-357570, a probe equipped with an intercalating fluorescent unit that has a nucleic acid-intercalating group selected among known compounds including flavine, porphyrin, quinone, and metallocene, a probe equipped with an intercalating, specific base-discriminating fluorescent unit (base-discriminating fluorescent nucleobase) that includes a nucleotide derivative as disclosed in Japanese Patent Laid-Open Gazette No. 2004-166522 and No. 2004-168672, or a probe equipped with a fluorescent unit that includes a self quenching group mutually associable to be quenched by association as disclosed in Japanese Patent Laid-Open Gazette No. 2002-281978.

The 'probe equipped with a unit having a signal change' is preferably a probe equipped with a unit having a fluorescence signal change. The 'fluorescence signal change' may be any one of a shift in fluorescence wavelength, an increase in fluorescence intensity, and a decrease in fluorescence intensity or any combination of two or all of these changes.

Typical examples of the 'unit having a fluorescence signal change' are the intercalating fluorescent unit and the specific base-discriminating fluorescent unit mentioned above. These units contains a fluorochrome intercalator that has a fluorescence signal change corresponding to each base to be hybridized. For example, pyrene, anthracene, and naphthalene are usable as the fluorochrome intercalator. The fluorescent intercalator may alternatively be prepared by binding a known fluorescent substance to a known intercalator. In this application, various aromatic dye molecules, such as acridine orange, proflavine, ethidium bromide, and actinomycin D, are usable as the known intercalator. Available examples of the known fluorescent substance include fluorescein isothiocyanate (FITC) and rhodamine derivatives.

Carbon chains and polymer chains are usable as a linker for binding the intercalator to the pyrimidine base or the purine base. The binding site of the pyrimidine base or the purine base with the intercalator is arbitrarily selected among non-substituted carbon sites. The binding site of the pyrimidine base is either the $4^{th}$ site or the $5^{th}$ site, and the binding site of the purine base is either the $7^{th}$ site or the $8^{th}$ site.

The intercalating fluorescent unit and the base-discriminating fluorescent unit are able to substituting a nucleotide corresponding to preset one base or preset 2 through10 bases included in the nucleic acid probe. The whole contents of Japanese Patent Laid-Open Gazette No. 2004-166522, No. 2004-168672, No. 2002-281978, and International Application No.PCT/JP2005/004703 and International Application No. PCT/JP2004/016602 are incorporated in the specification hereof by reference.

Any of the probes having the fluorescent units described above has a fluorescence signal change. The probe of the present invention preferably includes one of base-discriminating fluorescent nucleobase units expressed by Formulae (1) through (20) given below as nucleotide derivatives or nucleoside derivatives. The use of the base-discriminating fluorescent nucleobase unit lowers the labeling error and the background to reduce the overall measurement

error and allows clear discrimination between matching and mismatching. The probe having the base-discriminating fluorescent nucleobase unit is thus effectively used for detection of single nucleotide polymorphisms (SNPs) and other polymorphisms affecting the constitution, the drug responsiveness, and the drug sensitivity and for detection of gene mutations (including chimera genes caused by, for example, chromosome translocation) affecting the disease diagnosis, prognostic expectation, and the disease incidence estimation. The nucleobase units of Formulae (1) through (4) are adenine-discriminating fluorescent nucleobase units. The nucleobase units of Formulae (5) through (8) are thymine/uracil-discriminating fluorescent nucleobase units. The nucleobase units of Formulae (9) through (12) are guanine-discriminating fluorescent nucleobase units. The nucleobase units of Formulae (13) through (16) are cytosine-discriminating fluorescent nucleobase units. The nucleobase units of Formulae (17) through (20) are thymine/uracil-discriminating or cytosine-discriminating fluorescent nucleobase units.

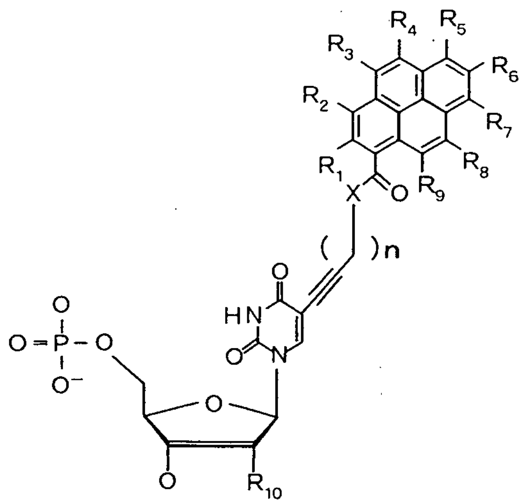

(1)

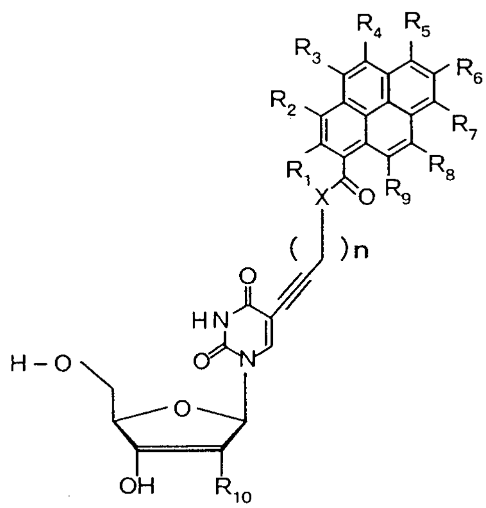

(2)

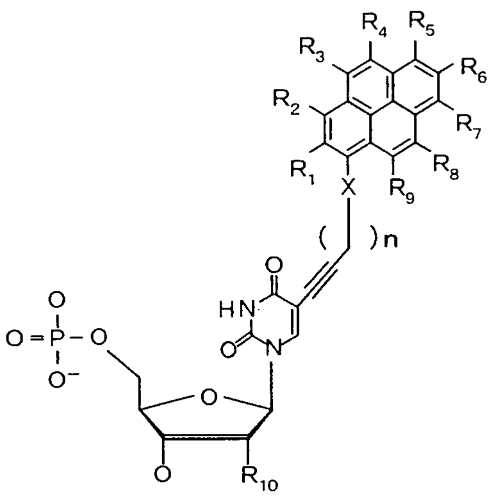

(3)

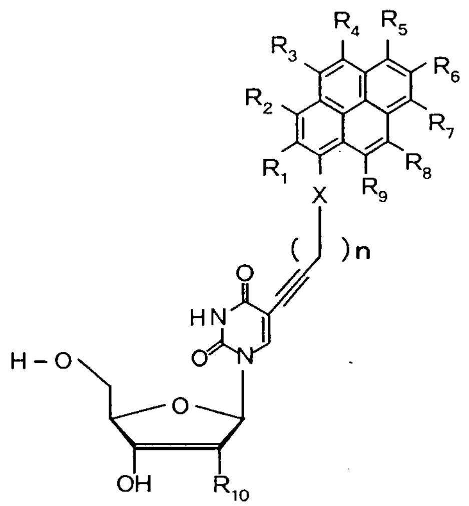

(4)

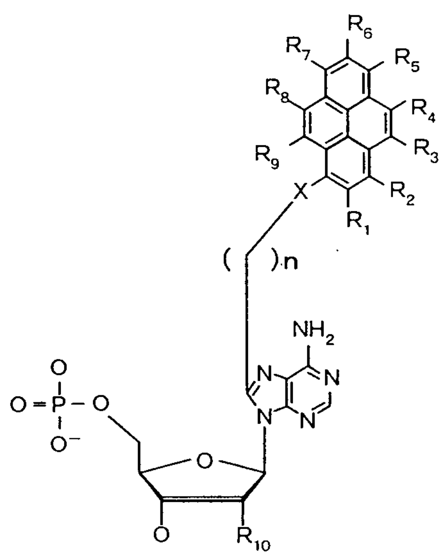

(5)

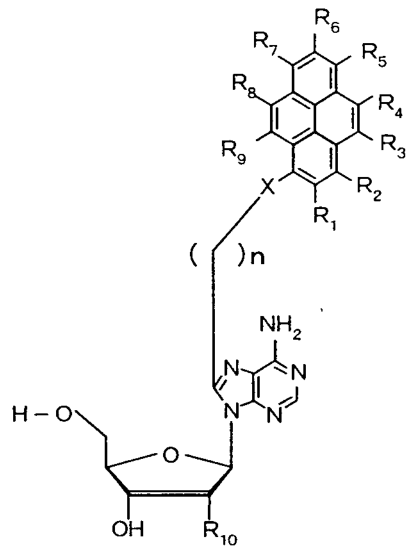

(6)

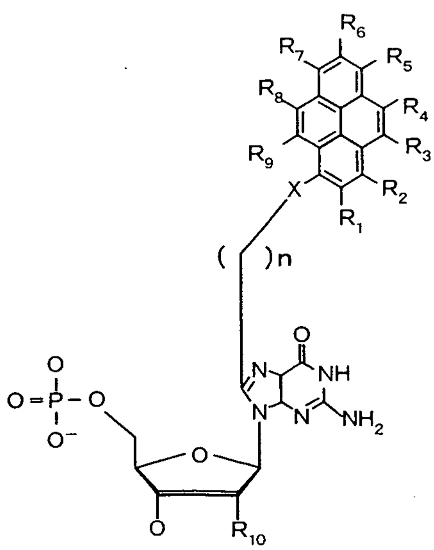

(7)

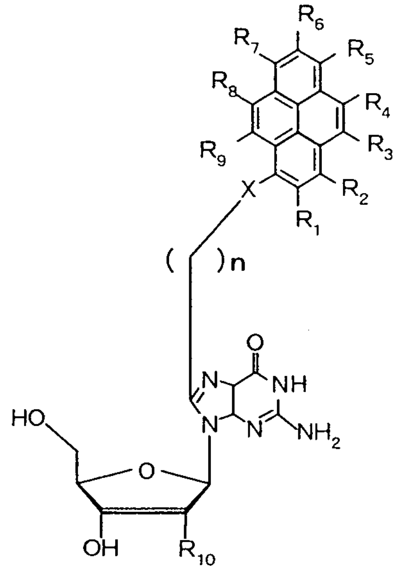

(8)

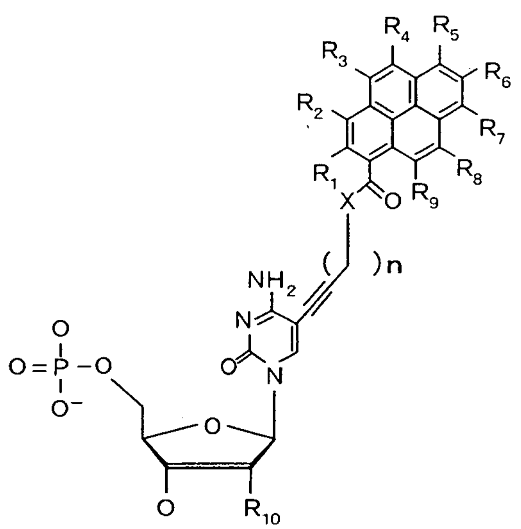

(9)

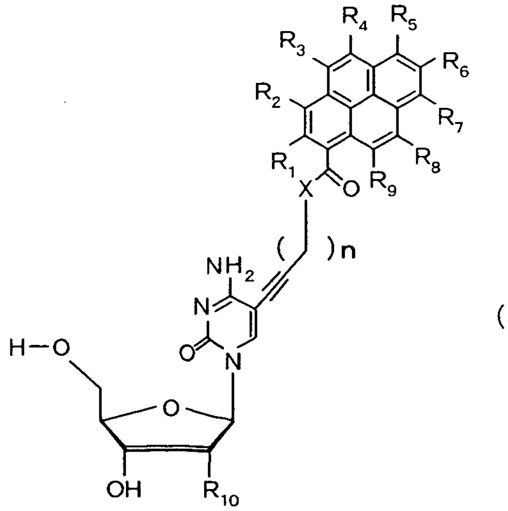

(10)

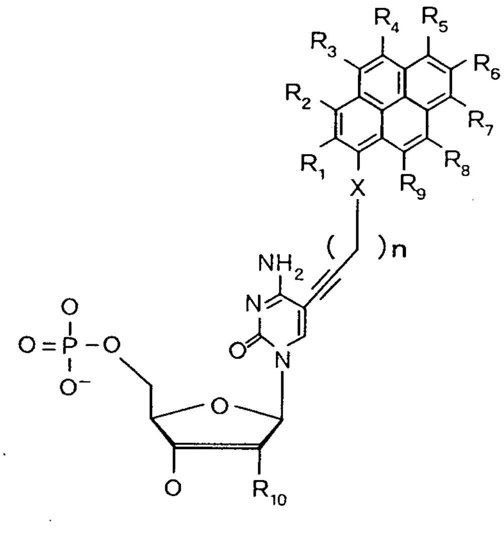

(11)

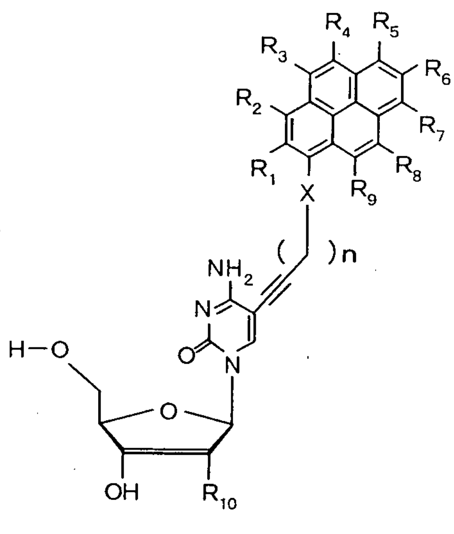

(12)

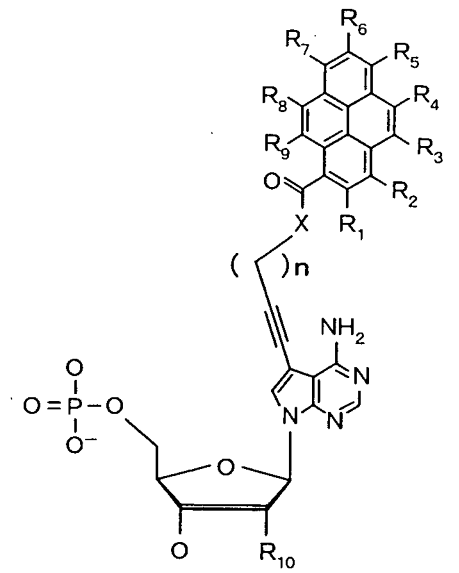

(13)

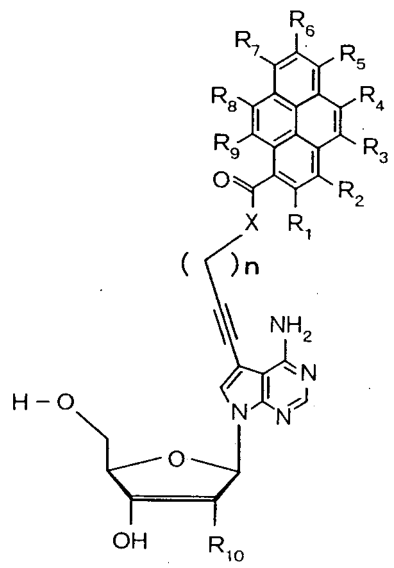

(14)

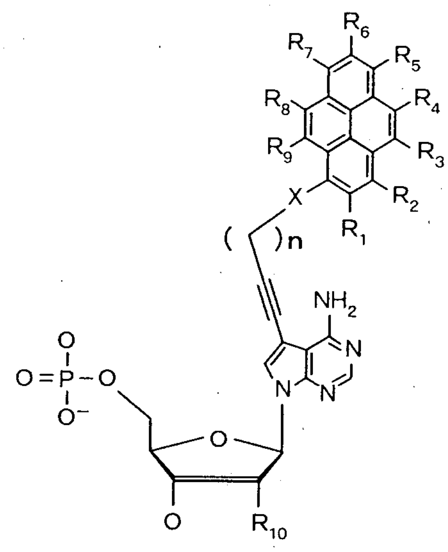

(15)

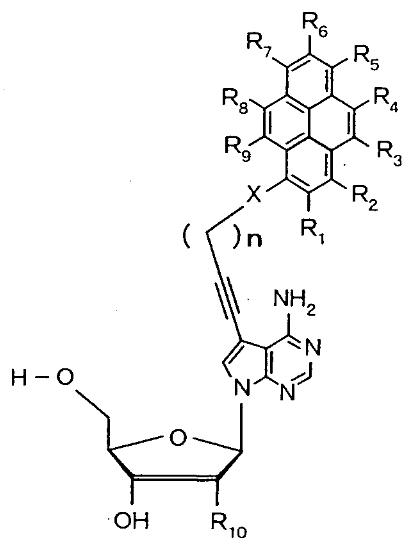

(16)

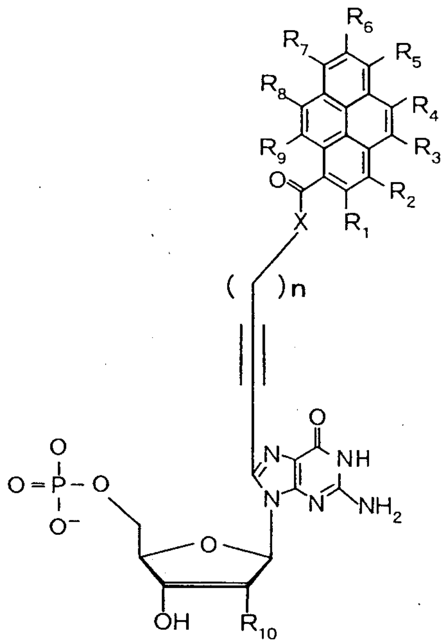

(17)

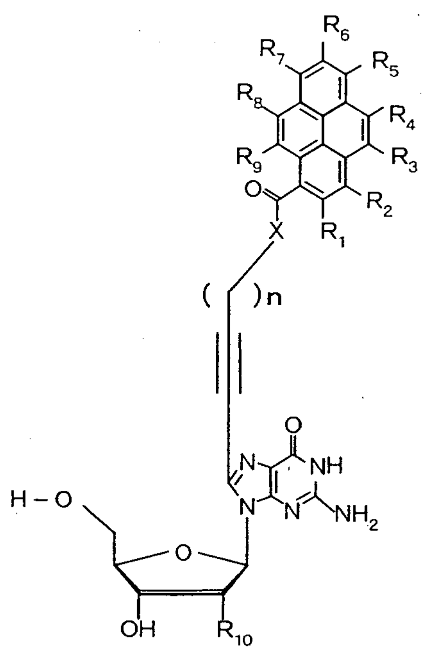

(18)

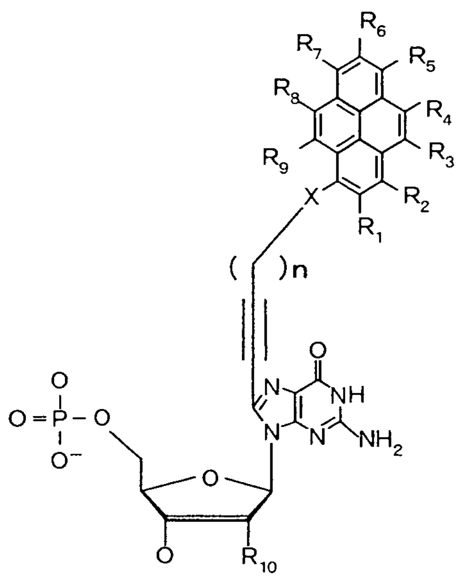

(19)

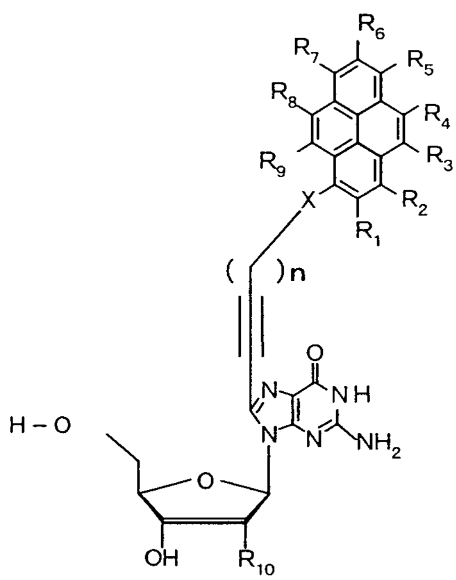

(20)

In Formulae (1) through (20) given above, each of $R_1$ through $R_9$ represents hydrogen atom or a substituent, $R_{10}$ represents hydrogen atom or hydroxyl group, and X denotes a linking group selected among imino (NH), oxy (O), thio (S), methylene ($CH_2$), and alkylamino groups. The integral number 'n' representing the length of alkylene chain is in a range of 0 to 5 for methylene and alkylamino groups as the linking group X and is in a range of 1 to 5 for imino, oxy, and thio groups as the linking group X. The substituents of $R_1$, $R_2$, $R_3$, $R_4$, $R_5$, $R_6$, $R_8$, and $R_9$ may be halogen atoms, oxygen-containing groups, nitrogen-containing groups, sulfur-containing groups, and hydrocarbon groups and heterocyclic groups having such atoms and element-containing groups. More specifically, typical examples of the substituents include halogen atoms, alkoxy groups, ester groups, amino groups, substituted amino groups, nitro groups, amide groups, cyano groups, carbamate groups, ureido groups, thiol groups, thioether groups, and thioester groups. $R_1$, $R_2$, $R_3$, $R_4$, $R_5$, $R_6$, $R_8$, and $R_9$ can not be simultaneously hydrogen atoms. Any adjacent pair among $R_1$, $R_2$, $R_3$, $R_4$, $R_5$, $R_6$, $R_8$, and $R_9$ may be mutually bonded to form a phenyl group or a substituted phenyl group.

Groups expressed by Formulae (21) through (27) given below may be used for the linking group X in Formulae (3) through (8), (11), (12), (15), (16), (19), and (20) given above. The linking group expressed by X is, however, not essential but may be omitted according to the requirements. In this case, the pyrimidine base or the purine base may be bonded directly to the fluorochrome intercalator via a linker.

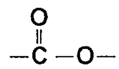 (21)

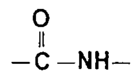 (22)

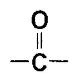 (23)

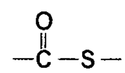 (24)

-O- (25)

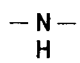 (26)

-s- (27)

The probe of the invention includes: (a) a characteristic detectable region where a characteristic on a base sequence of a sample is detectable as an object of detection; and (b) a stationary detectable region where a stationary sequence of the sample, which is located in proximity to the characteristic on the base sequence detected in the characteristic detectable region (a), is detectable. The stationary detectable region (b) represents any one of (1) an area with no detection of mutation, (2) an area estimated to have no mutation or have a high potential for no mutation, and (3) an area confirmed to have no mutation or have a high potential for no mutation. The probe has individually different signal changes induced by hybridization in the characteristic detectable region (a) and by hybridization in the stationary detectable region (b).

This arrangement of the invention enables one single probe to efficiently gain a subject signal corresponding to a target sequence included in a sample as an object of detection, as well as a control signal usable for signal correction and quantitative determination. A signal change induced by hybridization in the stationary detectable region (b) is made to arise regardless of matching or mismatching in the characteristic detectable region (a), for example, by adequately setting hybridization conditions. By taking advantage of mishybridization with the probe, the signal change in the stationary detectable region (b) represents the amount of a target nucleic acid contained in a sample supplied to the probe or the array with the probe fixed thereon. The control signal usable as the internal standard on the same probe desirably enhances the accuracy of measurement.

The characteristic on the base sequence of the sample detected in the characteristic detectable region (a) may be, for example, mutation, single nucleotide polymorphisms, or a specific chimera, such as a breakpoint of a chimera gene caused by chromosome translocation. The characteristic detectable region (a) has a base-discriminating fluorescent nucleobase for discriminating one or multiple specific bases included in the characteristic on the base sequence. The stationary sequence detected in the stationary detectable region (b) is not specifically restricted as mentioned above but is one or multiple stationary bases in proximity to the specific bases. The stationary detectable region (b) has a base-discriminating fluorescent nucleobase for discriminating the one or multiple stationary bases. The stationary sequence is preferably determined by taking into account each subject individual or a group including each subject individual, for example, a family group, a racial group, or an ethnic group.

The stationary sequence as the control is required to be detectable regardless of matching or mismatching of

the specific base. Preferably at least one base is interposed between the stationary base and the specific base.

For example, the acetaldehyde dehydrogenase 2 (ALDH2) gene has single nucleotide polymorphisms A/G at an $11^{th}$ site in a base sequence shown in Fig. 1. For detection of the polymorphisms, the probe is designed to have a first base-discriminating fluorescent nucleobase corresponding to a major genotype in the polymorphisms at the $11^{th}$ site and a second base-discriminating fluorescent nucleobase corresponding to an stationary base at a $6^{th}$ site with no observation of the polymorphisms.

The probe shown in Fig. 2 includes a polymorphism detectable region and a stationary base detectable region individually having first and second base-discriminating fluorescent nucleobases. A sample is exposed to the probe to be hybridized with the stationary base detectable region and induce fluorescence even in an incomplete pairing state in the polymorphism detectable region. Under such conditions, a resulting fluorescence signal derived from the second base-discriminating fluorescent nucleobase represents the amount of the supplied sample (ALDH2 gene). The polymorphism detectable region does not have fluorescence derived from the first base-discriminating fluorescent nucleobase in the case of unpairing to a target base as the object of detection, while having fluorescence derived from the first base-discriminating fluorescent nucleobase in the case of pairing to the target base. The ratio of the fluorescence signal derived from the first base-discriminating fluorescent nucleobase to the fluorescence signal derived from the second base-discriminating fluorescent nucleobase determines the content of a major genotype in the polymorphisms included in the sample, irrespective of the supplied amount of the sample. This arrangement readily identifies the polymorphisms without measuring the amount of the minor genotype. The probe of this structure easily determines the amount of a mutant sequence by taking advantage of the ratio of the fluorescence signal derived from the first base-discriminating fluorescent nucleobase to the fluorescence signal derived from the second base-discriminating fluorescent nucleobase in a standard solution including a known concentration of the mutant sequence. The fluorescence signal ratio in the standard solution may be specified in advance or may be measured for each assay of a sample.

The signal change detected in the characteristic detectable region (a) and the signal change detected in the stationary detectable region (b) are required to be clearly distinguishable from each other. For example, the signal changes in the respective regions (a) and (b) may have different fluorescence wavelengths.

The probe of the invention includes one or multiple units, each having a signal change by hybridization with a specific base included in a base sequence. All these units may have signal changes only by hybridization with specific bases included in a base sequence as the detection object in a sample. For example, the probe may have fluorescent units to detect individual sites in a chimera gene caused by, for example, chromosome translocation.

The present invention also provides a probe having only the stationary detectable region (b) for correction of the supplied amount of the sample. The combined fixation of this probe with a probe having only the characteristic detectable region (a) on one solid phase support similarly enables the correction and the analysis through measurement of respective signal changes and computation of the ratio of respective signals.

(Probe Carrier)

The probe carrier includes a solid phase support having a probe supported thereon. For example, the solid phase support is a substrate, and the probe carrier is an array. In another example, the solid phase support is particles as described later. A previously synthesized probe is fixed to the solid phase support by any suitable binding technique, for example, covalent bonding or electrostatic binding. The probe may be synthesized in situ on the surface of the solid phase support by photolithography or another suitable technique.

(Array)

The structure of an array 2 as one form of the probe carrier is shown in Fig. 3. The array 2 is a hybridization device that has a probe and is used for hybridization of a nucleic acid.

(Substrate)

In the array or the hybridization device 2, a substrate 4 has a nucleic acid fixation area 6 where at least a nucleic acid probe is fixed. The nucleic acid fixation area 6 includes or is designed to include one or multiple regions (spots) with fixation of nucleic acid probes. The fixation method or the fixation form adopted for fixation of the nucleic acid probe on the substrate 4 in the present invention is not specifically restricted. The fixation method and the fixation form may be adequately selected among various fixation technique and fixation forms known in the art at the moment of application of the present invention. The nucleic acid fixation area 6 of the substrate 4 preferably has a substantially flat shape including a minute three-dimensional shape. The substrate 4 may have one or multiple nucleic acid fixation areas 6 directly formed thereon or via a porous or any other suitable inclusion according to the requirements. The multiple nucleic acid fixation areas 6 formed on the substrate 4 may be separated from one another across hydrophobic partitions.

The shape of the substrate 4 is not specifically restricted. For example, the substrate 4 may be a flat plate. In another example, the substrate 4 may be a flat bottom of a concave body. The substrate 4 may be made of any suitable material selected among materials conventionally used for substrates and diversity of other materials. The available materials typically include glasses, silicon ceramics of, for example, silicon dioxide or silicon nitride, other ceramics, resins like silicones, polymethyl methacrylates, and poly(meth)acrylates, and metals like gold, silver, and copper. The substrate 4 may have an appropriate coating for gaining the desired surface properties. Especially preferable materials are glasses, silicones, and acrylic resins. Typical examples of the substrate 4 include a DNA chip with fixation of cDNA probes, a DNA microarray with fixation of cDNA probes, and a substrate for preparing a DNA microarray that currently has no fixation of cDNAs but is expected to have fixation of cDNAs.

(Cover Member)

The hybridization device 2 further includes a cover member 10 to cover over the substrate 4. The cover member 10 and the substrate 4 define a cavity 12 for hybridization reaction including the nucleic acid fixation area 6 on the substrate 4. The cover member 10 may be directly attached to the substrate 4 or may otherwise be attached to a substrate holder receiving and holding the substrate 4 therein to form the cavity 12. In the former application, the cover member 10 is attached to a flat plate or to the flat bottom of a concaved body as the substrate 4. In the latter application, the cover member 10 is attached to a substrate holder having a flat section or a concave section with a flat plate as the substrate 4 held therein.

The cavity 12 is a space that includes the nucleic acid fixation area 6 and has the capacity to be filled with a specific liquid for the hybridization reaction (hereafter referred to as 'hybridization liquid'). The cavity 12 preferably has a specific space height (or specific space depth) on the nucleic acid fixation area 6. The cover member 10 is preferably designed to hold an opposed area 14 facing the nucleic acid fixation area 6 of the substrate 4 across a predetermined distance from the substrate 4 even in the absence of the hybridization liquid. The cover member 10 of this design allows formation of the cavity 12 having the specific space height from the nucleic acid fixation area 6 by simply attaching the cover member 10 to the substrate 4 or the substrate holder.

At least the nucleic acid fixation area 6 of the substrate 4 and the opposed area 14 of the cover member 10 are exposed to the cavity 12. An additional surface required for shielding the cavity 12 from the outside is also exposed to the cavity 12, in addition to the nucleic acid fixation area 6 and the opposed area 14. The additional surface may be part of the substrate 4 or the cover member 10 or may be part or the whole of another element. The cavity 12 may have any suitable planar morphology but preferably has no projection or no corner because of their high potential for accumulation of the hybridization liquid. As described later, the cavity 12 may have an external projection or an extension that is sufficiently small and is formed in proximity to the side wall of curved configuration for totally preventing accumulation of the hybridization liquid. An elliptical shape shown in Figs. 3 and 4 and a circular shape are favorable examples of the planar morphology. The opposed area 14 of the cavity 12 may have a concave shape or a convex shape in the direction apart from the substrate 4 but preferably has planarity. The flat opposed area 14 facilitates formation of the cavity 12 that keeps a substantially fixed space height relative to the nucleic acid fixation area 6.

In the illustrated example of Fig. 4, the substrate 4 is a flat plate, and the cover member 10 has a spacer 8 of a predetermined height formed around the periphery of a flat plate element 10a including the opposed area 14. As shown in Fig. 3, the cover member 10 includes the flat plate element 10a having practically the same surface dimensions as those of the substrate 4 and a substantially flat plane on at least one side facing the substrate 4, and the spacer 8 formed around the periphery of the flat plate element 10a to be interposed between the substrate 4 and the flat plate element 10a. In another example, the cover member 10 may have a predetermined dome shape to cover over the nucleic acid fixation area 6. The cover member 10 of this structure may be a molded body of a polymer material. In the structure of the nucleic acid fixation area 6 placed in the bottom of a recess, the cover member 10 may be a flat plate attached to the upright circumferential wall of the recess. Some examples of this structure are the substrate 4 of a concave body having the nucleic acid fixation area 6 formed on the bottom of a recess, the substrate 4 having a peripheral side wall of a predetermined height around the nucleic acid fixation area 6, and the substrate 4 placed in the bottom of a substrate holder.

The spacer 8 may be made of any suitable material selected among acrylic resins, thermoplastic elastomers, natural ad synthetic rubbers, silicones, polyolefins, polyamides, polyimides, vinyl halides, and polycarbonates.

(Hydrophobic Region)

The hybridization device 2 has a hydrophobic region 16 in at least part of the area exposed to the cavity 12. The terminology 'hydrophobic' here represents a surface characteristic having at least the water repellency. The hydrophobic region 16 preferably has the higher water repellency than the water repellency of standard sodium silicate glass without any hydrophilic treatment. The water repellency is generally expressed by a water contact angle on the flat

surface. The water contact angle of the hydrophobic region in the present invention is not less than 30 degrees, preferably not less than 60 degrees, more preferably not less than 70 degrees, and most preferably not less than 90 degrees. The water contact angle represents a contact angle of a droplet placed on a level solid plane. The contact angle may be a static contact angle, a forward or rearward contact angle as the critical value, or a dynamic contact angle, but is preferably a static contact angle measured by the drop method.

There are three conventional techniques adopted for the drop method of measuring the static contact angle: (1) tangent method, (2) $\theta/2$ method, (3) three-point click method. The tangent method (1) adjusts the cursor of a reading microscope on the tangent of a droplet to directly measure the contact angle. The $\theta/2$ method (2) doubles the angle of a line between one end and an apex of a droplet to the solid surface to specify the contact angle. The three-point click method (3) clicks an apex of a droplet and two contact points of the droplet with the solid surface on a computer image and specifies the contact angle by image processing. Among these three methods, the method (2) or the method (3) is preferably applied to determine the contact angle in the present invention.

The hydrophobic region 16 is formed in at least part of the area exposed to the cavity 12 and is preferably provided on the cover member 10. The hydrophobic region 16 formed on the cover member 10 effectively enhances the signal intensity. The hydrophobic region 16 is preferably formed in the opposed area 14 of the cover member 10. More specifically, it is preferable to homogeneously form the hydrophobic region 16 over the whole opposed area 14 corresponding to substantially the whole nucleic acid fixation area 6. Although the opposed area 14 may have multiple discrete hydrophobic regions 16, it is preferable to form one continuous hydrophobic region 16 over substantially the whole opposed area 14. The whole exposed area of the cover member 10 exposed to the cavity 12 may form the hydrophobic region 16.

The cover member 10 may be fully made of a hydrophobic material to form the hydrophobic region 16. Alternatively only a specific area of the cover member 10 corresponding to the hydrophobic region 16 may be composed of the hydrophobic material. Otherwise the specific area of the cover member 10 may be subjected to certain surface treatment to give the hydrophobic surface characteristic (water repellency). Available examples of the hydrophobic material for the hydrophobic region 16 are polycarbonates, polyolefins including polyethylene and polypropylene, vinyl halides, polyamides, polyimides, acrylic resins, and fluorides and chlorides of these resins or polymers. The surface treatment to give the water repellency is, for example, chemical modification or mechanical processing of a certain material surface to be roughened and have the contact angle of not less than 90 degrees.

The distance between the nucleic acid fixation area 6 and the opposed area 14 in the cavity 12, that is, the space height over the nucleic acid fixation area 6 in the cavity 12 preferably has a coefficient of variation (standard deviation / average x 100 (%)) of not higher than 50%. The coefficient of variation in space height of not higher than 50% at the nucleic acid fixation area 6 desirably reduces a variation in signal intensity of the resulting hybridization product. The reduced variation of the signal intensity enables detection of high accuracy and attains the hybridization reaction of high reproducibility. The coefficient of variation in space height of not higher than 50% readily lowers the coefficient of variation in signal intensity to or below 20%. The coefficient of variation in space height is preferably not higher than 40%, more preferably not higher than 30%, and most preferably not higher than 20%. According to the inventors' findings, the coefficient of variation in space height of or below a predetermined level at the nucleic acid fixation area 6 in the cavity 12 has significant contribution to the evenness of the amount of the hybridization liquid (liquid thickness) per unit area of the nucleic acid fixation area 6.

The space height of the cavity 12 preferably has an average of not less than 15 $\mu$m. The average space height of not less than 15 $\mu$m desirably reduces a variation in signal intensity of the resulting hybridization product. More specifically the average space height is not less than 20 $\mu$m. The space height of not less than 20 $\mu$m in the cavity 12 allows a certain liquid thickness on the nucleic acid fixation area 6 and thus ensures convection of the hybridization liquid and diffusion of an object nucleic acid contained in the hybridization liquid, while controlling the potential effects of the exposed area to the cavity 12. The average space height preferably has an upper limit of 1000 $\mu$m. The parted area of the substrate 4 corresponding to the cavity 12 is preferably in a range of 1 $mm^2$ to 2000 $mm^2$.

The average space height and the coefficient of variation in space height in the cavity 12 may be determined by height-surface undulation measurement method described below.

(1) Measurement Sites

The measurement sites are on parting lines for dividing the cavity 12 defined by the cover member 10 or more specifically on the center line and on equally-divided parting lines of the cavity 12. In one example shown in Fig. 5(a), the measurement sites are on two parting lines that divide the cavity 12 into two equal parts both in the lateral direction and in the vertical direction. In another example shown in Fig. 5(b), the measurement sites are on six parting lines that divide the cavity 12 into four equal parts both in the lateral direction and in the vertical direction. In still another example shown in Fig. 5(c), the measurement sites are on ten parting lines that divide the cavity 12 into eight equal parts in the vertical direction and into four equal parts in the lateral direction.

(2) Measurement of Peripheral Height and Computation of Reference Height

A reference height (H) is determined first. The reference height (H) represents an average of height (peripheral height) from the surface of the substrate 4 including the nucleic acid fixation area 6 to the periphery of the cover member 10 corresponding to the circumferential part of the cavity 12 formed by attachment of the cover member 10 to face the nucleic acid fixation area 6 of the substrate 4. The peripheral height is measured at peripheral points on each parting line as shown in Fig. 5. Each parting line divides the cavity 12, so that the peripheral height is measured at two opposed peripheral points on each parting line. Namely the total number of measurement points for the peripheral height is equal to the number of parting lines x 2. The average of measurements of the peripheral height on all the parting lines is calculated and is defined as the reference height (H). For calculation of the average space height and the coefficient of variation in space height, the number of measurement points for the peripheral height is preferably not less than 4 or more specifically not less than 20.

(3) Measurement of Surface Undulation of Cover Member

The surface undulation of the cover member 10 is measured as a variation in surface convex and concave on each parting line relative to the circumference of a certain area on an outer surface of the cover member 10 (that is, a surface that does not face the substrate 4) corresponding to the opposed area 14. The maximum and the minimum of the surface undulation are measured on each parting line as the measurement traction. Namely there are two measurement points for the surface undulation on each parting line. The total number of measurement points for the surface undulation is equal to the number of parting lines x 2. For calculation of the average space height and the coefficient of variation in space height, the number of measurement points for the surface undulation is preferably not less than 4 or more specifically not less than 20.

(4) Measurement of Film Thickness

The film thickness of the cover member 10 represents the film thickness of the opposed area 14. The film thickness used here may be an average film thickness (Tave) of the opposed area 14 or may be film thicknesses (Tmax, Tmin) at measurement points of the maximum and the minimum of surface undulation, although the use of the average film thickness (Tave) is preferable. The film thickness is measured with any known measurement instrument, for example, a slide caliper.

(5) Computation of Space Height

Multiple space heights on each parting line are computable from these measured data. Maximum and minimum space heights on each parting line are determinable from the maximum and minimum surface undulations (MAX and MIN):

Maximum Space Height = Reference Height (H) + Maximum Surface Undulation (MAX) - Film Thickness (Tave or Tmax)

Minimum Space Height = Reference Height (H) + Minimum Surface Undulation (MIN) - Film Thickness (Tave or Tmin)

The maximum space height and the minimum space height obtained for all the parting lines are averaged to give an average space height, and standard deviation / average space height × 100 gives the coefficient of variation (%).

The peripheral heights of the cover member 10 at predetermined positions may be measured with a digital micrometer (Digimicro manufactured by Nikon Corporation), and the surface undulations of the cover member 10 may be measured with a surface texture and contour measuring instrument (Surfcom manufactured by Tokyo Seimitsu Co., Ltd).

The volume of the cavity 12 is preferably in a range of 0.1 μl to 2000 μl and is more preferably in a range of 1 μl to 1000 μl.

A specific portion of the cover member 10 including the opposed area 14 preferably has the optical transparency to enable the external observation of the inside of the cavity 12. The specific portion including the opposed area 14 preferably has an average thickness of not less than 300 μm. The average thickness of not less than 300 μm well controls the coefficient of variation in signal intensity of the resulting hybridization product. The average thickness of not less than 350 μm is more preferable. The upper limit of the average thickness is not specifically defined but is preferably not greater than 3000 μm, since the excessive thickness leads to an excess heat capacity and may cause an uneven temperature distribution in the cavity under heating.

(Other Structural Features)

The cover member 10 further has an opening 20 for injection of the hybridization liquid. There are preferably two or more openings 20, and it is desirable that at least one of the openings 20 is open in the vicinity of the contour for defining the cavity 12 in the cover member 10. This arrangement of the opening 20 prevents the hybridization liquid injected into the cavity 12 from retaining on the inner wall of the cavity 12 but facilitates diffusion of the hybridization liquid over the whole cavity 12. In the preferable structure, the openings 20 are formed along the contour of the cavity 12. More specifically, the openings 20 are formed as outward extensions from the inner wall of the cavity 12. In the illustrated structure of Fig. 3, two circular openings 20 in the cover member 10 are located at both ends of the elliptic cavity 12 in the longitudinal direction and are formed to be partially projected as extensions from the respective end walls of the cavity 12. This design of the openings 20 ensures sufficient diffusion of the hybridization liquid injected through the openings 20 over the whole cavity 12. The openings 20 are sealed with adequate sealing members.

The cover member 10 used for the hybridization device 2 is manufactured by combining the spacer 8 with the flat plate element 10a as the main body of the cover member 10 via a sealing layer 5. The sealing layer 5 may be an adhesive or binding layer for bonding the flat plate element 10a to the spacer 8. Attachment of multiple spacers 8 for parting adjacent space divisions to one cover member 10 readily defines multiple cavities 12 between the substrate 4 and the cover member 10.

The cover member 10 may not be an assembled body but may be an integrally molded resin body. The cover member 10 preferably has an adhesive or binding layer at a specific site for attachment to the substrate 4 or to the substrate holder. The adhesive layer is desirably protected by a detachable sheet. Another application of the invention is a hybridization reaction kit including the cover member 10 and the substrate 4. Fixation of a nucleic acid probe to the substrate 4 of this hybridization reaction kit gives a desired effective nucleic acid array. The cover member 10 may be provided separately from the substrate 4 or the substrate holder to be attachable to the substrate 4 or the substrate holder as occasion demands. The cover member 10 may be bonded to the substrate 4 or the substrate holder or may be molded as an integral body with the substrate 4 or the substrate holder. The cover member 10 may be detachably attached to the substrate 4 or the substrate holder for the convenience of cleaning and signal detection.

The specific portion of the cover member 10 including the opposed area 14 may have elastic deformability. The specific portion of the cover member 10 or the whole cover member 10 may be made of an elastically deformable material. Application of a gas pressure or mechanical external force to the opposed area 14 elastically deforms the specific portion or the whole cover member 10 to stir the hybridization liquid in the cavity 12.

An exposed side of the opposed area 14 of the cover member 10 exposed to the cavity 12 (that is, a side facing the substrate 4) may have concaves and/or convexes. These concaves and/or convexes give the complicated flow of the hybridization liquid and raise the stirring efficiency of the hybridization liquid in the cavity 12, thus enhancing the hybridization efficiency. The concaves and/or convexes may be formed integrally with the opposed area 14 of the cover

member 10 or may be obtained by application of a film or sheet with undulated surface on the side of the cover member 10 facing the substrate 4. The dimensions of the concaves and/or convexes are not specifically restricted but are set according to the space height of the cavity 12. The concaves and/or convexes may have hydrophobic regions.

(Other Probe Carriers)

A probe carrier of another structure uses particles of the solid phase support, instead of the substrate or flat plate of the solid phase support. The particles may be in a conventional spherical shape, such as beads, or may otherwise be in any of various shapes, for example, needle-like or amorphous. In this application, one probe having probe identification information represented by, for example, a color, fluorescence, a mark, a figure, a letter or character, or a pattern is fixed to each particle of the solid phase support. The probe identification information is correlated in advance to the type of the probe. In response to detection of a signal change induced by hybridization on each particle, a probe with probe identification information fixed to the particle is related to a sample DNA. In the flat plate of the solid phase support, the probe identification information on each probe represents the position of the probe on the solid phase support. The available materials for the particles of the solid phase support are similar to those for the flat plate of the solid phase support and include glasses, silicon ceramics of, for example, silicon dioxide or silicon nitride, other ceramics, resins like silicones, polymethyl methacrylates, and poly(meth)acrylates, and metals like gold, silver, and copper. The particles of the solid phase support may have appropriate coating for gaining the desired surface properties.

A probe carrier of still another application has a liquid-permeable solid phase support or a porous solid phase support in the form of either the flat plate or the particles. The solid phase support having liquid permeability or porosity expands a fixation area of a probe DNA and a contact area with a sample DNA, thus enabling high sensitive detection. The liquid-permeable solid phase support may have any structure that allows permeation of a liquid from one face to the other face of the solid phase support, for example, a filter-like structure or a porous structure.

(Hybridization Method of Nucleic Acid)

The hybridization reaction with the hybridization device 2 described above is performed according to the conventional procedure. The hybridization process using the hybridization device 2 first attaches the cover member 10 with a sealing layer on the side facing the substrate 4 to a DNA microarray as the substrate 4 via the sealing layer, injects a hybridization liquid prepared by a preset method through the two openings 20, seals the two openings 20 with sealing members, and keeps the DNA microarray 4 with the cover member 10 stationary at temperature of not lower than 25˚C and not higher than 80˚C for a preset time period.

The hybridization device 2 performs the hybridization reaction in the cavity 12, thus ensuring efficient hybridization. The hybridization device 2 has the hydrophobic region 16 in at least part of the exposed area to the cavity 12. The presence of the hydrophobic region 16 promotes convection of the hybridization liquid and diffusion of an object nucleic acid contained in the hybridization liquid in the cavity 12 without application of any external force to the substrate 4, for example, stirring, vibration, abrasion, or jet flow, to accelerate the hybridization reaction and enhance the efficiency of the hybridization reaction. The hybridization device 2 including the cover member 10 is preferably applied to the hybridization method that performs the hybridization reaction in the stationary state, as well as to various test methods including the hybridization process. Even in the stationary state, the hybridization device 2 has the sufficient effects of accelerating the hybridization reaction. The hybridization device 2 ensures the hybridization result of high reproducibility by reducing or even eliminating an operator-oriented variation caused by difference in handling operation of the substrate 4 and the cover member 10 and an external environment-based variation caused by, for example, the levelness of the hybridization device 2 for the stationary hybridization reaction and the magnitude of the external force.

The probe of the invention has a signal change induced only by the hybridization. The signal change is thus detectable immediately after the hybridization without any washing step, that is, in the presence of unhybridized DNAs mixed with the probe. This arrangement effectively eliminates an error derived from the washing step and ensures detection of high accuracy.

Controlling the space height and its coefficient of variation of the cavity 12 defined by the substrate 4 and the cover member 10 of the hybridization device 2 and regulating the thickness of the opposed area 14 of the cover member 10 reduce the variation in signal intensity of a hybridization product and enable signal detection of high accuracy. The simple control of the structure and the dimensions of the cavity 12 attains the effect of reducing the variation in signal intensity of the hybridization product without any complicated technique conventionally adopted for the same purpose. Such control of the structure and the dimensions of the cavity 12 also has the thermal buffer effect, which substantially equalizes the amount of the hybridization liquid per unit area of the nucleic acid fixation area 6.

The presence of the concaves and/or convexes on the side of the opposed area 14 of the cover member 10 facing the substrate 4 ensures the desired convection of the hybridization liquid in the stationary state and enhances the efficiency of the hybridization reaction.

The hybridization method may have a stirring step to stir the hybridization liquid in the cavity 12 and enhance the efficiency of hybridization. The hydrophobic region 16 may be provided in at least part of the cavity 12. The aqueous liquid stirred in the cavity 12 is repelled by the hydrophobic region 16. This accelerates the movement of the hybridization liquid in the cavity 12 and accordingly attains the higher efficiency of hybridization.

In the stirring step, the hybridization liquid in the cavity 12 may be forcibly stirred in a temporary, intermittent, or continuous manner. The movement of the hybridization device 2 including the substrate 4 is effective for stirring the hybridization liquid in the cavity 12. For example, the substrate 4 and the relevant members for defining the cavity 12 may be rotated, swirled, seesawed, reciprocated, turned upside down, or moved by combination of any two or more of such actions. When the opposed area 14 of the cover member 10 is made of an elastically deformable material, deformation of the opposed area 14 by an external force stirs the hybridization liquid in the cavity 12. In one example, a roller or another rotating member may be moved with rotation on the opposed area 14. In another example, a pressing member may be moved with application of pressure on the opposed area 14. Such active stirring may be continued throughout the hybridization process or may be performed intermittently or only in part of the hybridization process.

For effectively stirring the hybridization liquid in the cavity 12, a gas insoluble in the hybridization liquid (for example, the air or an inert gas like nitrogen) is preferably present in the cavity 12 separately from the hybridization liquid. While the cavity 12 is kept stationary, the gas in the cavity 12 is generally retained in a fixed position. The hybridization reaction does not vigorously proceed in this gas retention area (gas accumulation). Application of an external force to move the hybridization liquid in the cavity 12 in the presence of the gas desirably promotes the movement of the hybridization liquid in the cavity 12 and thus accelerates the hybridization reaction.

Hybridization of a probe carrier without any cavity 12 is occasionally performed according to the properties of the solid phase support (for example, the substrate or the particles, the porosity, the liquid permeability). The solid phase support of a substrate without liquid permeability, for example, a glass substrate, performs the hybridization reaction with supply of a sample solution onto the surface of the solid phase support as the conventional procedure. The solid phase support of particles performs the hybridization reaction while being soaked or suspended in a sample solution. The solid phase support of a flat plate with liquid permeability performs the hybridization reaction while being soaked in a sample solution or being impregnated with a sample solution. The probe carrier having the solid phase support of any of such properties enables detection of a signal change immediately after the hybridization reaction without removal of the sample solution by washing.

Another application of the invention is accordingly an array kit including a substrate 4 that has a nucleic acid fixation area with fixation of one or multiple nucleic acid probes, and a cover member 10 defining a cavity that includes the nucleic acid fixation area and allows storage of a hybridization liquid for hybridization of a nucleic acid. The array kit preferably has a hydrophobic region that is provided in at least part of an exposed area exposed to the inside of the cavity, for example, in an opposed area of the cover member facing the nucleic acid fixation area. In the array kit of this structure, the cover member 10 defines the cavity to allow the easy and efficient hybridization reaction of the nucleic acid. The cover member 10 may be integrated with the substrate 4 or a substrate holder via an adhesive or a binding agent. The cover member 10 may be detachably attached to the substrate 4 or the substrate holder.

Examples

Some examples of the invention are described below for the better understanding of the invention. These examples are only illustrative and are not restrictive in any sense.

Example 1

[Synthesis of Oligodeoxyribonucleotide Probe with Base-discriminating Fluorescent Nucleobases (Nucleotide Derivatives N1, N2, and N3)]

Among probes in Table 1 given below, all probes other than probes 2 and 7, that is, probes 1, 3 - 5, and 6 having specific sequences, were synthesized with preset base-discriminating fluorescent nucleobases.

(Probes 1, 4, 5)

Three fluorescent base-containing oligodeoxyribonucleotides were synthesized with an adenine-discriminating fluorescent nucleobase N1 ($^{AMPy}$U) disclosed in Patent No. WO2004/058793. The syntheses of these three oligodeoxyribonucleotides were in conformity with the conventional phosphoroamide method with Applied Biosystems 3400 DNA synthesizer. The resulting base sequences obtained by the syntheses are given below. The 5' terminals of these base sequences were amino-modified, and a spacer C12, 12-(4-monomethoxytritylamino) dodecyl-(2-cyanoethyl)- (N,N-diisopropyl) phosphoramidite, was introduced into the respective 5' terminals.

(Structural Formula of Fluorescent Base N1)

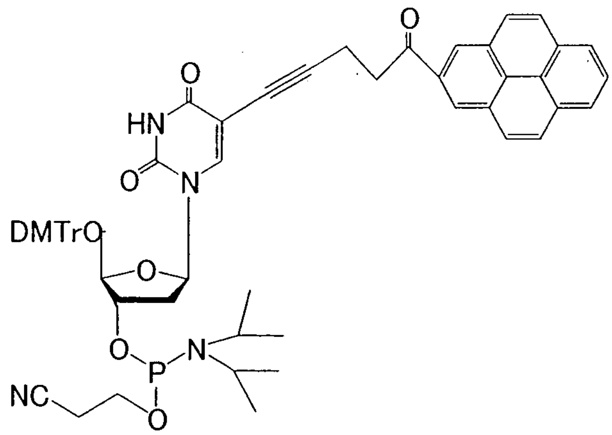

(Probe 3)

A fluorescent base-containing oligodeoxyribonucleotide was synthesized with adenine-discriminating fluorescent nucleobases N1 ([AMPy]U) and N2 ([Py]U) disclosed in Patent No. WO2004/058793. The synthesis of this oligodeoxyribonucleotide was in conformity with the conventional phosphoroamide method with Applied Biosystems 3400 DNA synthesizer. The resulting base sequence obtained by the synthesis is given below. The 5' terminal of the base sequence was amino-modified, and a spacer C12, 12-(4-monomethoxytritylamino) dodecyl-(2-cyanoethyl)- (N,N-diisopropyl) phosphoramidite, was introduced into the 5' terminal.

(Structural Formula of Fluorescent Base N2)

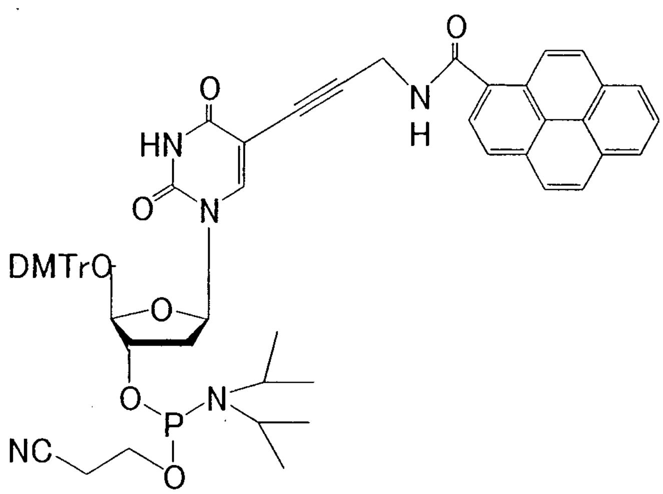

(Probe 6)

A fluorescent base-containing oligodeoxyribonucleotide was synthesized with a cytosine-discriminating fluorescent nucleobase N3 ([Py]C) disclosed in Patent No. WO2004/058793. The synthesis of this oligodeoxyribonucleotide was in conformity with the conventional phosphoroamide method with Applied Biosystems 3400 DNA synthesizer. The resulting base sequence obtained by the synthesis is given below. The 5' terminal of the base sequence was amino-modified, and a spacer C12, 12-(4-monomethoxytritylamino) dodecyl-(2-cyanoethyl)- (N,N-diisopropyl) phosphoramidite, was introduced into the 5' terminal.

(Structural Formula of Fluorescent Base N3)

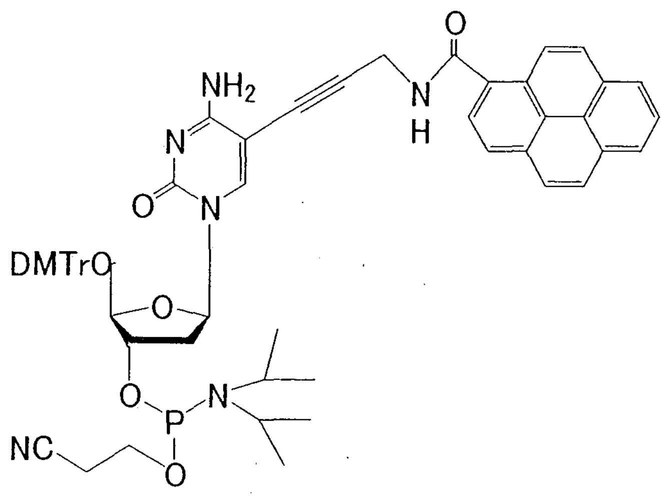

Table 1

| Probe | | Base Sequence (5' →3') | Sequence No. |
|---|---|---|---|
| No. | Detection Object | | |
| 1 | ALDH2 major genotype | NH2-SpacerC12-TTTTCACTT $^{AMPy}$U AGTGTATGCC | 1 |
| 2 | ALDH3 major genotype | NH2-SpacerC12-TTTTCACTTT AGTGTATGCC | 2 |
| 3 | ALDH4 major genotype | NH2-SpacerC12-TTTTCACTT $^{AMPy}$U AGTG $^{Py}$U ATGCC | 3 |
| 4 | b3a2 breakpoint proximity in bcr/abl gene | NH2-spacerC12-TGAAGGGCTT $^{AMPy}$U TGAACTCTG | 4 |
| 5 | b3 proximity in normal bcr gene | NH2-spacerC12-CAGTACAGAT $^{AMPy}$U TGAACTCTG | 5 |
| 6 | ALDH2 minor genotype | NH2-SpacerC12-TTTTCACTT $^{Py}$C AGTGTATGCC | 6 |
| 7 | ALDH3 minor genotype | NH2-SpacerC12-TTTTCACTTCAGTGTATGCC | 7 |

The probes 1, 2, and 3 were used for detection of the major genotype in the polymorphisms of ALDH2 (acetaldehyde dehydrogenase 2) gene, whereas the probes 6 and 7 were used for detection of the minor genotype in the polymorphisms of ALDH2 gene. The probe 4 was used for detection of a b3a2 breakpoint in the bcr/abl gene of Philadelphia chromosome of chronic myeloid leukemia (CML). The probe 5 was used for detection of a sequence b3 in the normal bcr gene on the human 9$^{th}$ chromosome (that is, a normal sequence in proximity to the b3a2 breakpoint of the bcr/abl gene) (see Table 1).

Each of these synthesized oligonucleotides was extracted from the solid phase support with aqueous ammonium, was placed in an Eppendorf tube, and was heated at 55°C for 8 hours for deprotection. The resulting aqueous solutions of the oligonucleotides were purified by high-performance liquid chromatography (600, Waters).

After the purification, removal of the solvent under the reduced pressure with a freeze dryer gave the objective oligonucleotides.

Example 2

[Preparation of DNA Microarray with Fixation of Oligodeoxyribonucleotides as Probes]

The probes 1, 3, 4, 5, and 6, the probe 2 with the amino-modified 5' terminal, and the probe 7 (manufactured by Nihon Gene Research Laboratories Inc.) with the amino-modified 5' terminal were spotted on a Codelink substrate manufactured by Amersham Bioscience Corp. The probe concentration of each spot solution was 50 pmol/μl, and the quantity of each spot was 150 pl. The substrate had 5 spots of each of these seven probe solutions.

The substrate with the spots of the respective probes was incubated overnight at the temperature of 20˚C and the relative humidity of 75%. A blocking solution having the following composition was regulated to have the concentration of 0.1% SDS by further addition of a 10% SDS solution. After incubation, the substrate was soaked in this solution mixture for 15 minutes. The substrate was washed with sterilized water twice, was washed with a 4xSSC 0.1%SDS solution (50˚C) for 30 minutes, and was washed again with sterilized water once. The substrate was then soaked in boiled water (98˚C) for 2 minutes and was washed with sterilized water twice. The substrate was centrifuged at 800 rpm and was dried. This completed a DNA microarray.

| Composition of Blocking Solution | |
|---|---|
| 1M Tris(pH8.0) | 20 ml |
| 99% Ethanol Amine | 0.6 ml |
| 10% SDS | 2 ml |
| Sterilized Water | 177.4 ml |
| Total | 200 ml |

Example 3

[Preparation of Sample DNAs]

Samples 1 to 13 shown in Table 2 were prepared as hybridization samples. The samples 1 to 6 were 20 mer synthetic DNAs corresponding to the homozygous major genotype, the homozygous minor genotype, and the heterozygous genotype of the ALDH2 gene. The samples 7 to 9 were 189 mer PCR-amplified samples (single stranded) corresponding to the homozygous major genotype, the homozygous minor genotype, and the heterozygous genotype of the ALDH2 gene. The samples 10 to 13 were 129 mer synthetic DNAs corresponding to specific regions in the b3a2 breakpoint proximity of the bcr/abl gene and in the b3 proximity of the bcr gene.

Table 2

| Sample | | Base Sequence (5' →3') | 5'Terminal | Length | Sequence No. |
|---|---|---|---|---|---|
| No. | Genotype | | | | |
| 1 | ALDH2 homozygous major | GGCATACACTAAAGTGAAAA | Non-labeled | 20mer | 8 |
| 2 | ALDH2 homozygous minor | GGCATACACTGAAGTGAAAA | Non-labeled | 20mer | 9 |
| 3 | ALDH2 heterozygous | Mixture of equal aliquots of above two base sequences | Non-labeled | 20mer | 8.9 |
| 4 | ALDH2 homozygous major | GGCATACACTAAAGTGAAAA | Cy3-labeled | 20mer | 8 |
| 5 | ALDH2 homozygous minor | GGCATACACTGAAGTGAAAA | Cy4-labeled | 20mer | 9 |
| 6 | ALDH2 heterozygous | Mixture of equal aliquots of above two base sequences | Cy5-labeled | 20mer | 8,9 |
| 7 | ALDH2 homozygous major | TATGATGTGTTTGGAGCCCAGTCACCCTTTGGTGGCTACAAGATGTCGG<br>GGAGTGGCCGGGAGTTGGGCGAGTACGGGCTGCAGGCATACACTAAAG<br>TGAAAACTGTCACAGTCAAAGTGCCTCAGAAGAACTCATAAGAATCAT<br>GCAAGCTTCCTCCCTCAGCCATTGATGGAAAGTTCAGCAAGATC | Non-labeled | 189mer | 10 |
| 8 | ALDH2 homozygous minor | TATGATGTGTTTGGAGCCCAGTCACCCTTTGGTGGCTACAAGATGTCGG<br>GGAGTGGCCGGGAGTTGGGCGAGTACGGGCTGCAGGCATACACTGAAG<br>TGAAAACTGTCACAGTCAAAGTGCCTCAGAAGAACTCATAAGAATCAT<br>GCAAGCTTCCTCCCTCAGCCATTGATGGAAAGTTCAGCAAGATC | Non-labeled | 189mer | 11 |
| 9 | ALDH2 heterozygous | Mixture of equal aliquots of above two base sequences | Non-labeled | 189mer | 10,11 |
| 10 | b3 proximity in normal ber gene | ATGATGAGTCTCCGGGGCTCTATGGGTTTCTGAATGTCATCGTCCACTC<br>AGCCACTGGATTTAAGCAGAGTTCAAATCTGTACTGCACCCTGGAGGT<br>GGATTCCTTTGGGTATTTTGTGAATAAAGCAA | Non-labeled | 129mer | 12 |
| 11 | b3a2 breakpoint proximity in ber/abl gene | ATGATGAGTCTCCGGGGCTCTATGGGTTTCTGAATGTCATCGTCCACTC<br>AGCCACTGGATTTAAGCAGAGTTCAAAAGCCCTTCAGCGGCCAGTAGC<br>ATCTGACTTTGAGCCTCAGGGTCTGAGTGAAG | Non-labeled | 129mer | 13 |
| 12 | b3 proximity in normal bcr gene | ATGATGAGTCTCCGGGGCTCTATGGGTTTCTGAATGTCATCGTCCACTC<br>AGCCACTGGATTTAAGCAGAGTTCAAATCTGTACTGCACCCTGGAGGT<br>GGATTCCTTTGGGTATTTTGTGAATAAAGCAA | Cy3-labeled | 129mer | 12 |
| 13 | b3a2 breakpoint proximity in bcr/abl gene | ATGATGAGTCTCCGGGGCTCTATGGGTTTCTGAATGTCATCGTCCACTC<br>AGCCACTGGATTTAAGCAGAGTTCAAAAGCCCTTCAGCGGCCAGTAGC<br>ATCTGACTTTGAGCCTCAGGGTCTGAGTGAAG | Cy4-labeled | 129mer | 13 |

The 20 mer and 129 mer synthetic DNAs were obtained from Nihon Gene Research Laboratories Inc. and from Sigma Genosys, Sigma-Aldrich Japan K.K. The samples 7 and 8 as the 189 mer PCR-amplified samples were synthesized from primers shown in Table 3 according to the following procedure. A thermal cycler GeneAmp PCR System 9700 (Applied Biosystems Japan Ltd.) for PCR, and the PCR products were identified by an electrophoresis apparatus (Bio-Rad Laboratories).

Table 3

| Sample | Primer* | Primer Sequence(5'→3') | Product Length | Annealing Temperature | GC | Sequence No. |
|---|---|---|---|---|---|---|
| 7** | F(non-modified) | TAT GAT GTG TTT GGA GCC | 189bp | 50˚C | 44% | 14 |
| | R(5'-biotinylated) | GAT CTT GCT GAA CTT TCC | | | | 15 |
| 8*** | F(non-modified) | TAT GAT GTG TTT GGA GCC | 189bp | 50˚C | 44% | 16 |
| | R(5'-biotinylated) | GAT CTT GCT GAA CTT TCC | | | | 17 |

F and R respectively denote the forward direction and the reverse direction in the primer.
Invitrogen CS0DM00Y123 was used as the genome sample for PCR amplification of the major genotype.
Open Biosystems 4849418 was used as the genome sample for PCR amplification of the minor genotype.

PCR Amplification Conditions

The genome samples were individually amplified by PCR with Takara Ex Taq (Takara Bio Inc). The conditions of the PCR reaction are given below.
1. Preparation of Mixture

| | |
|---|---|
| Sterilized Water | 79.5 μl |
| PCR buffer (10X) | 10 μl |
| dNTP mixture (2.5 nM) | 8 μl |
| TAKARA Ex Taq (5 unit/pl) | 0.5 μl |
| Primer-F (100 pM) | 0.5 μl |
| Primer-R (100 μM) | 0.5 μl |
| Genome Sample | 1 μl |
| Total | 100 μl |

2. Thermal Cycle Reaction (96˚C for 30 seconds → 94˚C for 1 minute → annealing temperature for 1 minute → 72˚C for 2 minutes at 35 cycles → 72˚C for 20 minutes → temperature lowered to 4˚C)

The PCR-amplified major genotype sample and minor genotype sample were subjected to sequence analyses. According to the results of the sequence analyses, the amplified major genotype product and the amplified minor genotype product were identified to be 189 bp double-stranded DNAs respectively including objective sequences. The double-stranded samples were denatured with Streptavidin sepharose in the presence of NaOH to the single strands.

Each sample (10 pmol / 50 μl) thus obtained was dissolved in a 50mM phosphate buffer (pH 7.0) (50 μl) containing 0.1 M sodium chloride in a sample tube. The sample solution was heated with a 95˚C heat block for 2 minutes, was stood at room temperature for 5 minutes, was centrifuged, and was regulated to have a final concentration of 100 nM. Each resulting sample solution was used as a hybridization sample.

Example 4

[Manufacture of Hybridization Device (Chamber Type) with Cavity]

A hybridization device (chamber type) used for hybridization was manufactured in this example. The hybridization chamber has the DNA microarray prepared in Example 2, a polycarbonate spacer, and a glass (borosilicate glass) cover with small holes. As shown in Fig. 6, the spacer is a perforated sheet member that defines an elliptical region on a probe fixation area of the DNA microarray as a cavity of a preset depth. The cover closes the opening of the cavity provided on the DNA microarray and has small holes formed at the longitudinal ends of the cavity for supply of a hybridization liquid. The spacer was integrated with the DNA microarray having the probes fixed thereon, and the cover with the small holes was then attached to the spacer to cover over the opening of the cavity. The hybridization device thus assembled had the hybridization chamber with the small holes for supply of the hybridization liquid formed at both the ends along the longitudinal axis of the cavity. In the hybridization device, the cavity had the longer diameter of approximately 45 mm, the shorter diameter of approximately 15 mm, and the internal height of 500 μm. The glass cover had the thickness of 150 μm and the volume of approximately 400 μl. The hybridization device had the transparency to enable detection of a signal change (fluorescence emission) induced by hybridization with each DNA sample and was designed to have only a low level of spontaneous fluorescence that does not interfere with detection of the signal change. Hybridization experiments were performed with this chamber-type hybridization device. The results of hybridization were measured as fluorescence signals and were numerically analyzed for evaluation.

Example 5

[Evaluation of Hybridization with Hybridization Device of Example 4]

Example 5 showed evaluation for enhancement of fluorescence signals in hybridization with the hybridization device manufactured in Example 4. In Example 5, the cavity of the hybridization device of Example 4 was filled with the solution of the sample 1 prepared in Example 3 (the major genotype DNA sample of the non-labeled ALDH2 gene) and was kept stationary at 42°C for 16 hours for hybridization. The fluorescence signal from the hybridization product of the probe 1 was measured for evaluation. As a control, the same DNA sample was spotted (200 μl, each spot: circular shape of approximately 100 μm in diameter) on the DNA microarray prepared in Example 3, was covered with a glass cover, and was kept stationary at 42°C for 16 hours for hybridization (Comparative Example 5). The fluorescence signal from a hybridization product of the probe 1 in Comparative Example 5 was measured after washing and drying steps. The measured fluorescence signals were numerically analyzed according to a numerical analysis software program GenePix pro (Axon Instruments). Fig. 7 shows the characteristic structures of the hybridization devices used in Example 5 and in Comparative Example 5 as the control and the hybridization - fluorescence detection procedures in Example 5 and in Comparative Example 5. Table 4 shows the conditions of the hybridization and the fluorescence detection in Example 5 and in Comparative Example 5. The fluorescence signals were measured with a biochip reader (Applied Precision LLC). The results of the fluorescence detection are shown in Fig. 7.

Table 4

| | Probe No. | Sample No. | Hybridization Condition | Fluorescence Detection |
|---|---|---|---|---|
| Example 5 | 1 | 1 | With chamber | Fluorescence wavelength of $^{AMPy}$U: 460±25nm (excitation wavelength 360±20nm) |
| Comparative Example 5 | 1 | 1 | Without chamber | the same as above |

As shown in Fig. 7, the fluorescence signal in Example 5 was about 1.8 times as much as the fluorescence signal in Comparative Example 5. This proves the enhanced detection ability for the same DNA sample. The use of the hybridization device manufactured in Example 4 effectively enhances the hybridization efficiency. The hybridization - fluorescence detection procedure of Example 5 does not require the washing and drying steps in the hybridization - fluorescence detection procedure of Comparative Example 5 and thus desirably shortens the time required for washing and drying (by approximately 2 hours).

Example 6

Example 6 showed evaluation for detection of fluorescence signals from the non-labeled ALDH2 gene samples.

According to the same procedure as Example 5, the non-labeled DNA samples (samples 1 to 3) prepared in Example 3 were subjected to hybridization with the hybridization device of Example 4. The fluorescence signals of the respective hybridization products were measured. The variation CV in fluorescence signal among the multiple spots of each identical probe was computed for evaluation. As a control, the DNA samples labeled with the Cy3 fluorescent agent (samples 4 to 6) were hybridized, washed, and dried, and their fluorescence signals were measured (Comparative Example 6). Table 5 shows the conditions of the hybridization and the fluorescence detection in Example 6 and in Comparative Example 6. The fluorescence signals were measured with the biochip reader (Applied Precision LLC). The results of the fluorescence detection are shown in Figs. 8 and 9.

Table 5

| | Probe No. | Sample No. | Hybridization Condition | Fluorescence Detection |
|---|---|---|---|---|
| Example 6 | 1 | 1,2,3 | Example 5 | Fluorescence wavelength of $^{AMPy}$U: 460±25nm (excitation wavelength 360±20nm) |
| Comparative Example 6 | 2 | 4,5,6 | Comparative Example 5 | Cy3 fluorescence wavelength |

As clearly shown by the comparison between the graphs of Figs. 8 and 9, Comparative Example 6 of Fig. 9 had the greater variations CV and had difficulty in accurate fluorescence detection of the respective DNA samples labeled with the Cy3 fluorescent agent (samples 4 to 6). Example 6 of Fig. 8, on the other hand, had the smaller variations CV and enabled accurate fluorescence detection of the respective non-labeled DNA samples (samples 1 to 3). As mentioned above, the hybridization device manufactured in Example 4 had the chamber for the DNA microarray with fixation of the oligodeoxyribonucleotide probes containing the base-discriminating fluorescent nucleobases. This hybridization device ensures accurate detection of the single nucleotide polymorphisms of the ALDH2 gene even for the non-labeled DNA samples.

Example 7

Example 7 showed the stirring effect of the hybridization liquid, which was kept in the cavity of the hybridization device manufactured in Example 4, on the variation (CV) in fluorescence signal among the multiple spots of each identical probe in the DNA microarray. In Example 7, each of the three non-labeled long-chain DNA samples (samples 7 to 9) prepared in Example 3 was supplied into the chamber of the hybridization device of Example 4 (with fixation of the fluorescent nucleobase-containing oligodeoxyribonucleotide probes) and was hybridized at 42°C for 16 hours with stirring. The fluorescence signals of the respective hybridization products were measured. The variation CV in fluorescence signal among the multiple spots of each identical probe was computed for evaluation. Each of the DNA sample solutions in the cavity was stirred by rotating the hybridization device. As a control, the DNA microarray with each of the same DNA samples (samples 7 to 9) as those of Example 7 was hybridized at 42°C for 16 hours in the stationary state. The fluorescence signals of the respective hybridization products in Comparative Example 7 were measured. The variation CV in fluorescence signal among the multiple spots of each identical probe was computed for evaluation. Table 6 shows the conditions of the hybridization and the fluorescence detection in Example 7 and in Comparative Example 7. The fluorescence signals were measured with the biochip reader (Applied Precision LLC). The results of the fluorescence detection are shown in Fig. 10.

Table 6

| | Probe No. | Sample No. | Hybridization Condition | Fluorescence Detection |
|---|---|---|---|---|
| Example 7 | 1 | 7,8,9 | Stirring chamber | Fluorescence wavelength of $^{AMPy}$U: 460±25nm (excitation wavelength 360±20nm) |
| Comparative Example 7 | 1 | 7,8,9 | Keeping chamber stationary | the same as above |

As shown in Fig. 10, the operation of stirring the hybridization liquid in the hybridization device manufactured in Example 4 expanded the differences among the fluorescence signals of the respective samples. The stirring operation, on the

other hand, reduced the variation CV in fluorescence signal among the multiple spots of each identical probe from 6 through 8% to less than 5%. This proves the enhanced reliability of fluorescence detection. Namely the operation of stirring the hybridization liquid in the cavity improves the hybridization efficiency and the detection accuracy.

Example 8

Example 8 showed evaluation for detection of the ALDH2 gene samples with the probe having a different fluorescent base for detection of an internal standard in the stationary sequence of the oligodeoxyribonucleotide containing a fluorescent base for detection of a mutation site of the ALDH2 gene. In Example 8, each of the three non-labeled long-chain DNA samples (sample 7: homozygous major genotype sample of the ALDH2 gene, sample 8: homozygous minor genotype sample of the ALDH2 gene, sample9: heterozygous sample of the ALDH2 gene) prepared in Example 3 was supplied into the chamber of the hybridization device of Example 4 and was hybridized at 42°C for 16 hours with stirring by rotation of the hybridization device. The fluorescence signal derived from the fluorescent base for detection of the mutation site and the fluorescence signal derived from the fluorescent base for detection of the internal standard in the hybridization product were measured for evaluation. Table 7 shows the conditions of the hybridization and the fluorescence detection in Example 8. The fluorescence signals were measured with the biochip reader (Applied Precision LLC). The results of the fluorescence detection are shown in Fig. 11.

Table 7

| | Probe No. | Sample No. | Hybridization Condition | Fluorescence Detection | |
|---|---|---|---|---|---|
| | | | | Mutation site | Internal standard |
| Example 8 | 3 | 7,8,9 | Stirring chamber | Fluorescence wavelength of $^{AMPy}U$: 460±25nm (excitation wavelength 360±20nm) | Fluorescence wavelength of $^{Py}U$: 400nm (excitation wavelength |

The graph of Fig. 11 shows the signal intensity of the fluorescent base for detection of the internal standard as the abscissa and the signal intensity of the fluorescent base for detection of the mutation site as the ordinate. While the signal intensity of the fluorescent base for detection of the internal standard was almost the same among the respective samples 7 through 9, the signal intensity of the fluorescent base for detection of the mutation site varied corresponding to the mutations in the respective samples 7 through 9. The ratio of the fluorescent base signal for detection of the mutation site to the fluorescent base signal for detection of the internal standard substantially represented the ratio of the mutations (homozygous major, homozygous minor, and heterozygous) in the respective samples 7 through 9. Based on these experimental results, the signal of the base-discriminating fluorescent nucleobase introduced into the stationary sequence of the probe 3 is usable as the signal of the internal standard. The probe having the base-discriminating fluorescent nucleobase for detection of the internal standard can determine the relative amount of a target site in a sample, regardless of a variation in amount of the sample supplied to the array, a variation in amount of the labeling, and a variation in amount of DNAs per gene. The conventional procedure requires both a major genotype probe (for example, the probe 1) and a minor genotype probe (for example, the probe 6) for detection and evaluation of a mutation gene. The procedure of Example 8, on the other hand, enables detection of the mutation gene with only one probe without the effect of mishybridization.

Example 9

Example 9 showed evaluation for detection of the bcr/abl gene samples as the causative gene of chromic myeloid leukemia with the oligodeoxyribonucleotide probes containing the base-discriminating fluorescent nucleobases. In Example 9, the non-labeled DNA samples prepared in Example 3 (sample 10: normal sequence sample in proximity to b3 of the bcr gene, sample 11: sequence sample in proximity to the b3a2 breakpoint of the bcr/abl gene) were hybridized with the hybridization device of Example 4. The fluorescence signals of the respective hybridization products were measured for evaluation. As a control, the DNA samples labeled with the Cy3 fluorescent agent (sample 12: normal sequence sample in proximity to b3 of the bcr gene, sample 13: sequence sample in proximity to the b3a2 breakpoint of the bcr/abl gene) were hybridized with the probes 4 and 5, washed, and dried, and the fluorescence signals of the respective hybridization products were measured for evaluation. Table 8 shows the conditions of the hybridization and the fluorescence detection in Example 9 and in Comparative Example 9. The fluorescence signals were measured with

the biochip reader (Applied Precision LLC). The results of the fluorescence detection are shown in Figs. 12 and 13.

Table 8

| | Probe No. | Sample No. | Hybridization Condition | Fluorescence Detection |
|---|---|---|---|---|
| Example 9 | 4, 5 | 10, 11 | Example 5 | Fluorescence wavelength of $^{AMPy}U$: 460±25nm (excitation wavelength 360±20nm) |
| Comparative Example 9 | 4, 5 | 12, 13 | Comparative. Example 5 | the same as above |

In Example 9 shown in Fig. 12, according to the hybridization results of the two DNA samples with the probe 4 specific for the b3a2 breakpoint of the bcr/abl gene, the sequence sample in proximity to the b3a2 breakpoint of the chimera bcr/abl gene (sample 11) had a significantly higher signal intensity. According to the hybridization results of the two DNA samples with the probe 5 for detection of the normal sequence of the bcr gene, the normal sequence sample of the bcr gene (sample 10) had a significantly higher signal intensity. In Comparative Example 9 shown in Fig. 13, however, there was no significant difference in signal intensity between the normal sequence sample of the bcr gene (sample 12) and the sequence sample in proximity to the b3a2 breakpoint of the chimera bcr/abl gene (sample 13) with regard to both the hybridization products with the probe 4 and with the probe 5.

There may be two probes having a common sequence and the potentials for mishybridization with two corresponding samples. Introduction of base-discriminating fluorescent nucleobases into respective high sequence-specific regions of these two probes (for example, regions in proximity to a breakpoint or non-common sequence regions) and measurement of fluorescence signals derived from these nucleobases enabled easy discrimination of the two samples. These two probes commonly have a sequence for identifying a sequence b3 in the bcr gene, and the two corresponding samples commonly have the sequence b3. The normal b3 sequence sample (sample 12) has a potential for mishybridization with the b3a2 probe (probe 4), whereas the b3a2 chimera sequence sample (sample 13) has a potential for mishybridization with the b3-proximity normal sequence probe (probe 5). The sample 10 and the sample 11 similarly have potentials for mishybridization with the probe 4 and mishybridization with the probe 5, respectively. The base-discriminating fluorescent nucleobases included in the probes 4 and 5, however, do not generate fluorescence in the case of such mishybridization. In Example 9, measurement of the fluorescence signals derived from the fluorescent nucleobases in the probes 4 and 5 can effectively detect only the completely matching DNA samples with the probes having the common sequence.

Example 10

[Evaluation of Gene Detection with BDF Probes Fixed to Solid Phase Support of Porous Particles]

Example 10 made evaluation for detection of the ALDH2 gene samples with a solid phase support of porous particles having individual fixation of the base-discriminating fluorescent nucleobase-containing ALDH2 gene homozygous major sequence probe (probe 1) and the base-discriminating fluorescent nucleobase-containing ALDH2 gene homozygous minor sequence probe (probe 6). Available materials for the solid phase support include inorganic compounds, synthetic polymer compounds, natural polymer compounds, and glasses. In Example 10, porous glass particles (average particle diameter: 3.1 μm, pore size: 30 nm) activated and degassed by surface plasma irradiation were soaked in a 1 wt% aqueous solution of a silane agent LS-2940 (Shin-Etsu Chemical Co. , Ltd.) having epoxy functional groups at room temperature for 1 hour for coupling. The solid phase support of the porous glass particles was completed by washing out the excess silane agent with water.

The porous glass particles were added individually to an aqueous solution of the probe 1 and to an aqueous solution of the probe 6 and were incubated for 16 hours to make covalent bonds of the respective probes 1 and 6 with the epoxy groups of the silane agent on the surface of the particles. The excess aqueous solutions were washed out with a PBS solution. The three non-labeled long-chain DNA sample solutions (samples 7 to 9, ALDH2 gene homozygous major genotype sample, ALDH2 gene homozygous minor genotype sample, and ALDH2 gene heterozygous genotypes ample) prepared in Example 3 were respectively supplied to an equal-amount mixture of the glass particles with fixation of the probe 1 and the glass particles with fixation of the probe 6 and were hybridized at 42°C for 15 minutes. The fluorescence signals derived from the fluorescent bases of the respective probes were measured for evaluation with a flow cytometer Cyto ACE-300 (manufactured by JASCO Cooperation) (excitation wavelength of 360±20 nm and fluorescence wavelength of 460±25 nm for the probe 1, excitation wavelength of 360±20 nm and fluorescence wavelength of 400 nm for the probe 6). The results of the measurement are shown in Fig. 14.

The fluorescence signal ratios were obtained corresponding to the ratios of the major genotypes to the minor genotypes of the respective samples as shown in Fig. 14. The solid phase support of the particles enabled both efficient hybridization and specific hybridization.

Example 11

[Evaluation of Gene Detection with BDF Probes Fixed to Solid Phase Support of Liquid-Permeable Flat Plate]

Example 11 made evaluation for detection of the ALDH2 gene samples with a solid phase support of liquid-permeable flat plate having fixation of both the base-discriminating fluorescent nucleobase-containing ALDH2 gene homozygous major sequence probe (probe 1) and the base-discriminating fluorescent nucleobase-containing ALDH2 gene homozygous minor sequence probe (probe 6). Available materials for the solid phase support include inorganic compounds, synthetic polymer compounds, natural polymer compounds, and glasses. In Example 11, a porous glass plate (pore size: 10 nm) activated and degassed by surface plasma irradiation was soaked in a 1 wt% aqueous solution of the silane agent LS-2940 (Shin-Etsu Chemical Co., Ltd.) having epoxy functional groups at room temperature for 1 hour for coupling. The solid phase support of the flat plate was completed by washing out the excess silane agent with water.

The probes 1 and 6 were spotted at preset positions on the glass plate by the inkjet technique and were incubated for 16 hours to make covalent bonds with the epoxy groups of the silane agent on the surface of the glass plate. The excess probes were washed out with a PBS solution. Each of the three non-labeled long-chain DNA sample solutions (samples 7 to 9, ALDH2 gene homozygous major genotype sample, ALDH2 gene homozygous minor genotype sample, and ALDH2 gene heterozygous genotypes ample) prepared in Example 3 was supplied to the glass plate. The glass plate impregnated with each of the sample solutions was hybridized at 42˚C for 15 minutes. While the glass plate was not washed but was filled with the sample solution, spot images of the respective samples were taken with a fluorescent microscope Olympus BX50 and a CCD camera Olympus M3204-C. The fluorescence signals derived from the fluorescent bases of the respective probes were measured for evaluation (excitation wavelength of 360±20 nm and fluorescence wavelength of 460±25 nm for the probe 1, excitation wavelength of 360+20 nm and fluorescence wavelength of 400 nm for the probe 6). The results of the measurement are shown in Fig. 15.

The fluorescence signal ratios were obtained corresponding to the ratios of the major genotypes to the minor genotypes of the respective samples as shown in Fig. 15. The solid phase support of the flat plate also enabled both efficient hybridization and specific hybridization.

The whole contents of the US preliminary patent application No. 60/435,995 filed on December 26, 2002, Japanese patent application No. 2003-314556 filed on September 5, 2003, the US preliminary patent application No. 60/523,318 filed on November 20, 2003, the international application PCT/JP2003/016602 filed on December 24, 2003, the US patent application No. 10/795,436 filed on March 9, 2004, and the international application PCT/JP2005/004703 filed on March 9, 2005 are incorporated in part of the specification hereof by reference.

The present application claims priority from the US preliminary patent application No. 60/643,603 filed on January 14, 2005 and the US preliminary patent application No. 60/660,224 filed on March 11, 2005, the contents of which are hereby incorporated by reference into this application.

Industrial Applicability

The technique of the invention is preferably applied to the industries of manufacturing detection devices of nucleic acids included in biological samples and the relevant industries utilizing the results of such detection.

Sequences

Sequence Numbers 1 to 7: Synthetic Nucleotides
Sequence Numbers 14 to 17: Synthetic Nucleotides

## Claims

1. An array, comprising:

   a substrate;
   a nucleic acid probe that is fixed to the substrate and is hybridized with a sample to have a signal change; and
   at least one cavity that is filled with a specific liquid containing the sample and causing the hybridization of the nucleic acid probe with the sample.

2. The array according to claim 1, wherein the at least one cavity is provided in a recess that is formed in the substrate and includes a fixation area of the nucleic acid probe on the substrate.

3. The array according to claim 1, the array further having a cover member.

4. The array according to claim 1 or 2, wherein the at least one cavity is provided in a hollow space of a cover member that is located at a certain height to face and cover over a fixation area of the nucleic acid probe on the substrate.

5. The array according to claim 4, wherein the cover member forms part of a vessel that holds the array therein.

6. The array according to any of claims 3 to 5, wherein the cover member is detachably attached to the substrate.

7. The array according to any of claims 3 to 6, wherein the cover member is fastened to the array via an adhesive or a binding agent.

8. The array according to any of claims 3 to 7, wherein the cover member has transparency to allow detection of the signal change caused by the hybridization of the nucleic acid probe with the sample.

9. The array according to any of claims 3 to 8, wherein the cover member has an inlet to fill the at least one cavity with the specific liquid for causing the hybridization.

10. The array according to any of claims 3 to 9, wherein an opposed area of the cover member facing a fixation area of the nucleic acid probe on the substrate has a thickness of not less than 300 $\mu$m.

11. The array according to any of claims 3 to 10, wherein an opposed area of the cover member facing a fixation area of the nucleic acid probe on the substrate is made of one or multiple materials selected among the group consisting

of glasses, polycarbonates, polyolefins, polyamides, polyimides, acrylic resins, fluorides of the acrylic resins, and polyvinyl halides.

**12.** The array according to any of claims 1 to 11, the array having a hydrophobic region in at least part of an exposed area exposed to inside of the cavity.

**13.** The array according to any of claims 3 to 12, wherein at least part of the cover member has a hydrophobic region.

**14.** The array according to claim 12 or 13, wherein the hydrophobic region is provided in an opposed area facing a fixation area of the nucleic acid probe on the substrate.

**15.** The array according to any of claims 12 to 14, wherein the hydrophobic region has a water contact angle of not less than 30 degrees.

**16.** The array according to any of claims 1 to 15, wherein the cavity has at least one of a height with a coefficient of variation of not higher than 50% and an average height of not less than 15 μm, where the height represents a distance between a fixation area of the nucleic acid probe on the substrate and an opposed area facing the fixation area.

**17.** The array according to any of claims 1 to 16, wherein the nucleic acid probe is hybridizable to have a fluorescence signal change that is any one or any combination of a shift in fluorescence wavelength, an increase in fluorescence intensity, and a decrease in fluorescence intensity.

**18.** The array according to claim 17, wherein the nucleic acid probe includes a base-discriminating fluorescent nucleobase.

**19.** The array according to any of claims 1 to 18, wherein the sample is an unlabelled nucleic acid.

**20.** The array according to any of claims 1 to 19, wherein the nucleic acid probe is in a range of 20 mer to 100 mer, and a detection accuracy of the signal change has a of not higher than 10% or preferably not higher than 5%.

**21.** The array according to any of claims 1 to 20, the array having at least one or multiple applications among constitution identification like single nucleotide polymorphisms, affected gene diagnosis as various gene mutations including gene chimeras, prognostic expectation, drug responsiveness detection, and drug resistance detection.

**22.** A hybridization method of a nucleic acid, the hybridization method comprising:

a hybridization step of filling the at least one cavity of the array according to any of claims 1 to 21 with the specific liquid containing the sample to cause the hybridization of the nucleic acid probe with the sample.

**23.** The hybridization method according to claim 22, wherein the hybridization step causes the hybridization of the nucleic acid probe with the sample while the array is kept stationary or while the specific liquid in the at least one cavity of the array is forcibly stirred in a temporary, intermittent, or continuous manner.

**24.** The hybridization method according to claim 22 or 23,
wherein the sample is an unlabelled nucleic acid.

**25.** The hybridization method according to any of claims 22 to 24, the hybridization method further having:

a detection step that follows the hybridization step and detects a signal change of the nucleic acid probe induced by the hybridization.

**26.** The hybridization method according to claim 25, wherein the detection step detects the signal change, while the specific liquid containing the sample is kept in the at least one cavity after the hybridization step.

**27.** The hybridization method according to claim 26, wherein the detection step detects the signal change after the hybridization step without any washing step.

**28.** A probe carrier for hybridization of a nucleic acid, the probe carrier comprising:

a solid phase support; and
a probe that is fixed to the solid phase support in an identifiable manner and is hybridized with a sample to have a signal change.

**29.** The probe carrier according to claim 28, wherein the solid phase support is either a flat plate or particles.

**30.** The probe carrier according to claim 28 or 29, wherein the solid phase support has either liquid permeability or porosity.

**31.** The probe carrier according to any of claims 28 to 30, wherein the solid phase support allows fixation of only one single type of the probe and has probe identification information selected among color, fluorescence, mark, figure, letter, character, and pattern.

**32.** The probe carrier according to any of claims 28 to 31,
wherein the probe is hybridizable to have a fluorescence signal change that is any one or any combination of a shift in fluorescence wavelength, an increase in fluorescence intensity, and a decrease in fluorescence intensity.

**33.** The probe carrier according to claim 32, wherein the probe includes a base-discriminating fluorescent nucleobase.

**34.** The probe carrier according to any of claims 28 to 33,
wherein the sample is an unlabelled nucleic acid.

**35.** The probe carrier according to any of claims 28 to 34, the probe carrier being used in a state that the probe carrier is soaked in or suspended in a specific liquid containing the sample.

**36.** A hybridization method of a nucleic acid, the hybridization method comprising:

a hybridization step of filling a cavity, which includes the probe carrier according to any of claims 28 to 35, with a specific liquid containing the sample to cause the hybridization of the probe with the sample.

**37.** The hybridization method according to claim 36, the hybridization method further having:

a detection step that follows the hybridization step and detects the signal change in the presence of the specific liquid containing the sample.

**38.** A probe for hybridization of a nucleic acid, the probe comprising:

(a) a characteristic detectable region where a characteristic on a base sequence of a sample is detectable as an object of detection; and
(b) a stationary detectable region where a stationary sequence of the sample, which is located in proximity to the characteristic on the base sequence detected in the characteristic detectable region (a), is detectable,

wherein the stationary detectable region (b) represents any one of (1) an area with no detection of mutation, (2) an area estimated to have no mutation or have a high potential for no mutation, and (3) an area confirmed to have no mutation or have a high potential for no mutation,
the probe having individually different signal changes induced by hybridization in the characteristic detectable region (a) and by hybridization in the stationary detectable region (b).

**39.** The probe according to claim 38, wherein each of the individually different signal changes induced by the hybridization in the characteristic detectable region (a) and by the hybridization in the stationary detectable region (b) is a fluorescence signal change that is any one or any combination of a shift in fluorescence wavelength, an increase in fluorescence intensity, and a decrease in fluorescence intensity.

**40.** The probe carrier according to claim 38 or 39, wherein the probe includes a base-discriminating fluorescent nucleobase.

**41.** The probe carrier according to any of claims 38 to 40, wherein the sample is an unlabelled nucleic acid.

**42.** A probe carrier for hybridization of a nucleic acid, the probe carrier comprising:

a solid phase support; and
the probe according to any of claims 38 to 41 that is fixed to the solid phase support.

**43.** The probe carrier according to claim 42, wherein the solid phase support is either a flat plate or particles.

**44.** The probe carrier according to claim 42 or 43, wherein the solid phase support has either liquid permeability or porosity.

**45.** The probe carrier according to any of claims 42 to 44, wherein the solid phase support allows fixation of only one single type of the probe and has probe identification information selected among color, fluorescence, mark, figure, letter, character, and pattern.

**46.** The probe carrier according to any of claims 42 to 45, the probe carrier being used in a state that the probe carrier is soaked in or suspended in a specific liquid containing the sample.

**47.** The probe carrier according to any of claims 42 to 46, wherein the probe is in a range of 20 mer to 100 mer.

**48.** The probe carrier according to any of claims 42 to 47, wherein a detection accuracy of the signal change has a CV of not higher than 10%.

**49.** The probe carrier according to any of claims 42 to 48, the probe carrier having at least one or multiple applications among constitution identification like single nucleotide polymorphisms, affected gene diagnosis as various gene mutations including gene chimeras prognostic expectation, drug responsiveness detection, and drug resistance detection.

**50.** A nucleic acid hybridization device, comprising:

the probe carrier according to any of claims 42 to 49; and
at least one cavity that is filled with a specific liquid for causing hybridization of the probe included in the probe carrier.

**51.** The nucleic acid hybridization device according to claim 50, the nucleic acid hybridization device having a hydrophobic region in at least part of an exposed area exposed to inside of the cavity.

**52.** The nucleic acid hybridization device according to claim 51, wherein the hydrophobic region has a water contact angle of not less than 30 degrees.

**53.** The nucleic acid hybridization device according to claim 51 or 52, wherein the hydrophobic region is made of one or multiple materials selected among the group consisting of glasses, polycarbonates, polyolefins, polyamides, polyimides, acrylic resins, fluorides of the acrylic resins, and polyvinyl halides.

**54.** The nucleic acid hybridization device according to any of claims 51 to 53, the nucleic acid hybridization device further having a cover member that covers over an opening of the at least one cavity.

**55.** The nucleic acid hybridization device according to claim 54, wherein the solid phase support is a substrate, and the cover member is detachably attached to the substrate.

**56.** The nucleic acid hybridization device according to claim 54 or 55, wherein the cover member has transparency to allow detection of the signal change caused by the hybridization of the probe with the sample.

**57.** The nucleic acid hybridization device according to any of claims 54 to 56, wherein an opposed area of the cover member facing a fixation area of the probe on the solid phase support has a thickness of not less than 300 $\mu$m.

**58.** The nucleic acid hybridization device according to any of claims 54 to 57, wherein at least part of the cover member has a hydrophobic region.

**59.** The nucleic acid hybridization device according to claim 58, wherein the hydrophobic region is provided in an opposed area facing a fixation area of the probe on the solid phase support.

**60.** The nucleic acid hybridization device according to any of claims 54 to 59, wherein the cavity has at least one of a height with a coefficient of variation of not higher than 50% and an average height of not less than 15 $\mu$m, where the height represents a distance between a fixation area of the probe on the solid phase support and an opposed area facing the fixation area.

**61.** A nucleic acid hybridization method, the nucleic acid hybridization method comprising:

a hybridization step of filling a cavity, which includes the probe carrier according to any of claims 42 to 49, with a specific liquid containing the sample to cause the hybridization of the probe with the sample.

**62.** The nucleic acid hybridization method according to claim 61, wherein the hybridization step causes the hybridization of the probe while the nucleic acid hybridization device is kept stationary or while the specific liquid in the cavity is forcibly stirred in a temporary, intermittent, or continuous manner.

**63.** The nucleic acid hybridization method according to claim 61 or 62, the nucleic acid hybridization method further having:

a detection step that follows the hybridization step and detects the signal change induced by the hybridization of the probe.

**64.** The nucleic acid hybridization method according to claim 63, wherein the detection step detects the signal change in the presence of the specific liquid containing the sample after the hybridization step.

**65.** The nucleic acid hybridization method according to claim 64, wherein the detection signal detects the signal change after the hybridization step without any washing step.

**66.** A probe for hybridization of a nucleic acid, the probe having a stationary detectable region where a stationary sequence is detectable with regard to a subject individual or with regard to a group including a subject individual, such as a family group, a racial group, or an ethnic group,
wherein the stationary detectable region represents any one of (1) an area with no detection of mutation, (2) an area estimated to have no mutation or have a high potential for no mutation, and (3) an area confirmed to have no mutation or have a high potential for no mutation,
the probe having a signal change induced by hybridization in the stationary detectable region.

**67.** The probe according to claim 66, wherein the signal change induced by the hybridization in the stationary detectable region of the stationary sequence is a fluorescence signal change that is any one or any combination of a shift in fluorescence wavelength, an increase in fluorescence intensity, and a decrease in fluorescence intensity.

FIG. 1

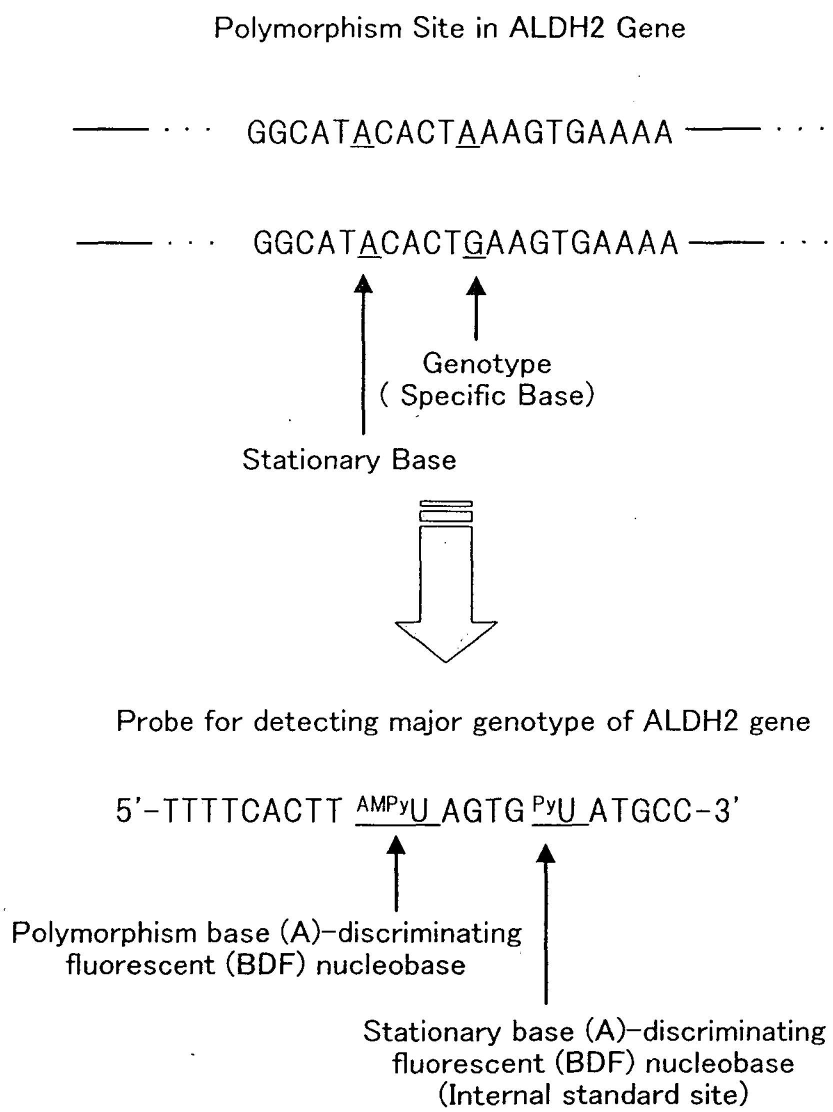
Polymorphism Site in ALDH2 Gene
GGCATACACTAAAGTGAAAA
GGCATACACTGAAGTGAAAA
Genotype
( Specific Base)
Stationary Base
Probe for detecting major genotype of ALDH2 gene
5'-TTTTCACTT AMPyU AGTG PyU ATGCC-3'
Polymorphism base (A)-discriminating
fluorescent (BDF) nucleobase
Stationary base (A)-discriminating
fluorescent (BDF) nucleobase
(Internal standard site)

FIG. 2

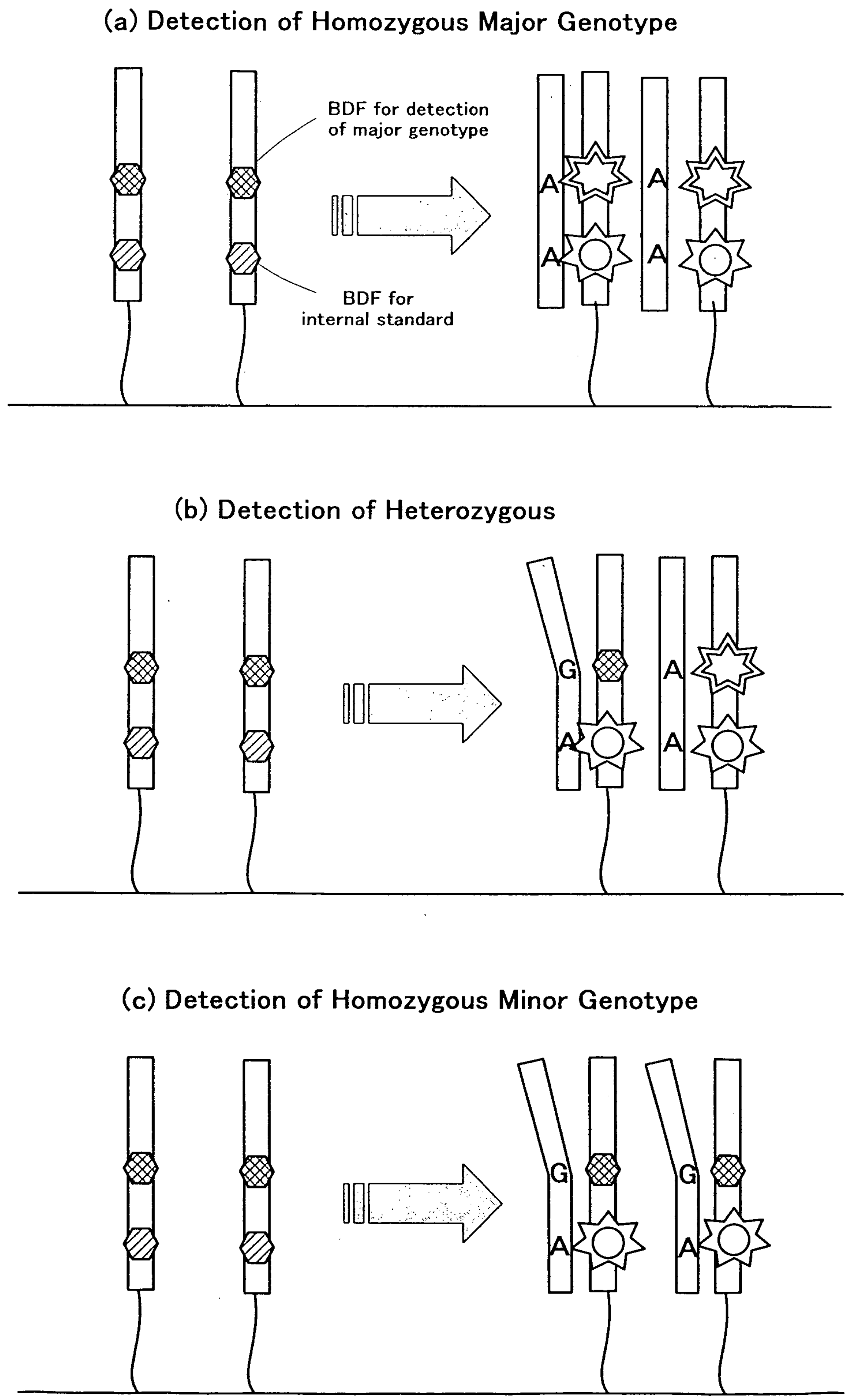
(a) Detection of Homozygous Major Genotype
BDF for detection of major genotype
BDF for internal standard
A
A
A
A
(b) Detection of Heterozygous
G
A
A
A
(c) Detection of Homozygous Minor Genotype
G
A
G
A

FIG. 3

(a)

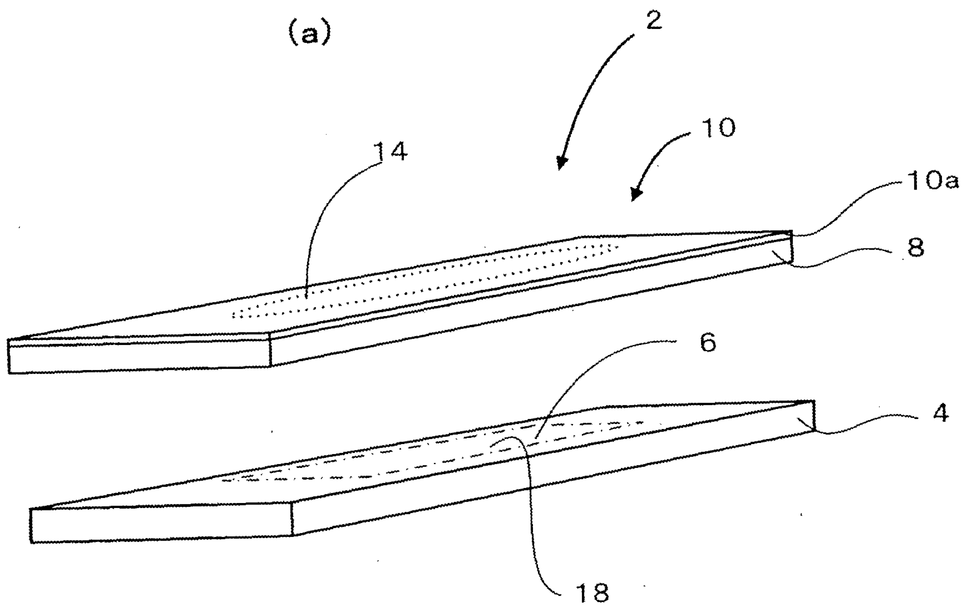


(b)

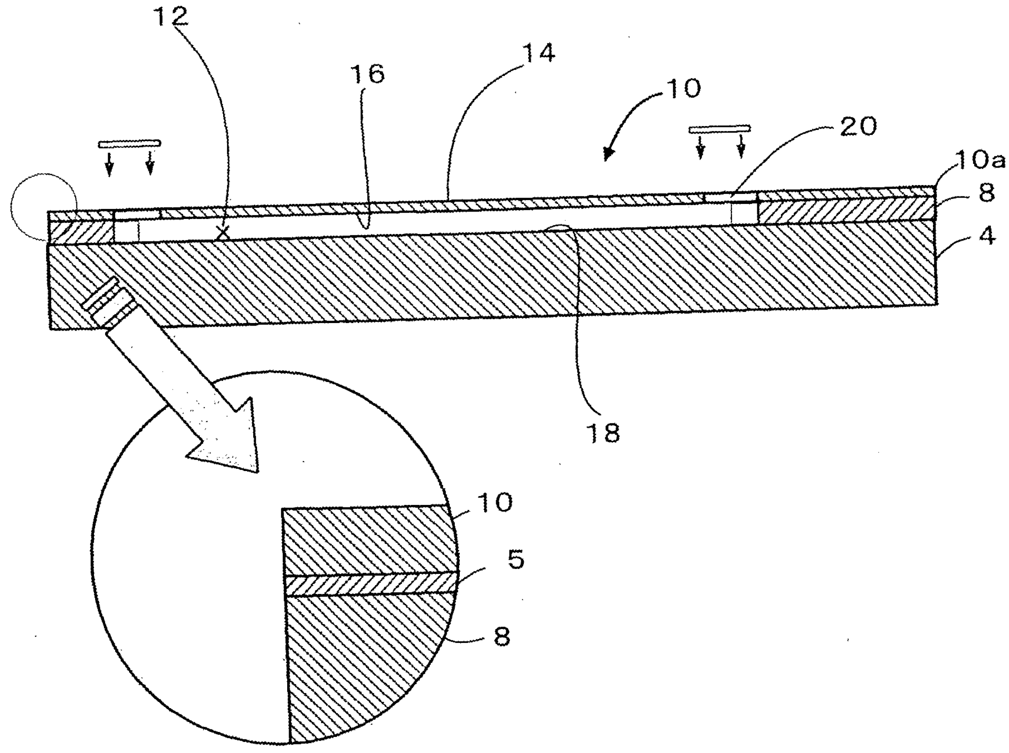

FIG. 4

(a)

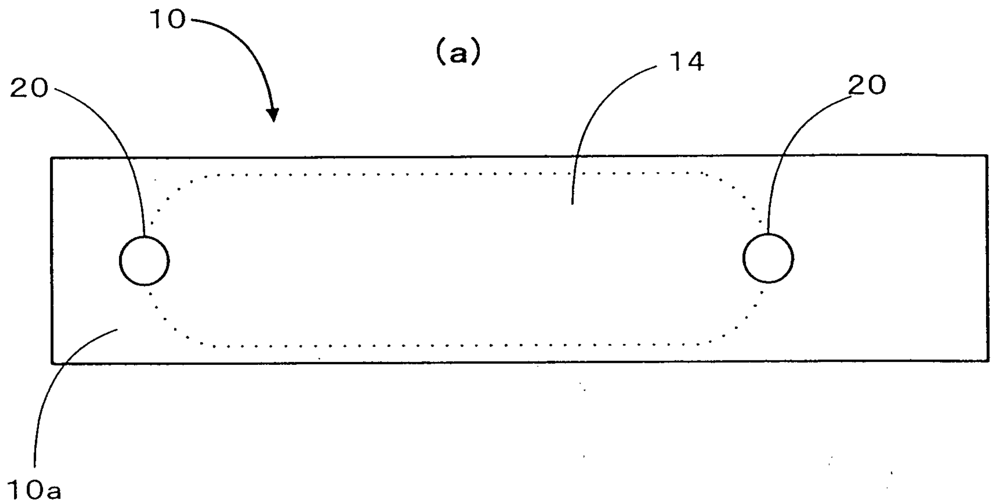


(b)

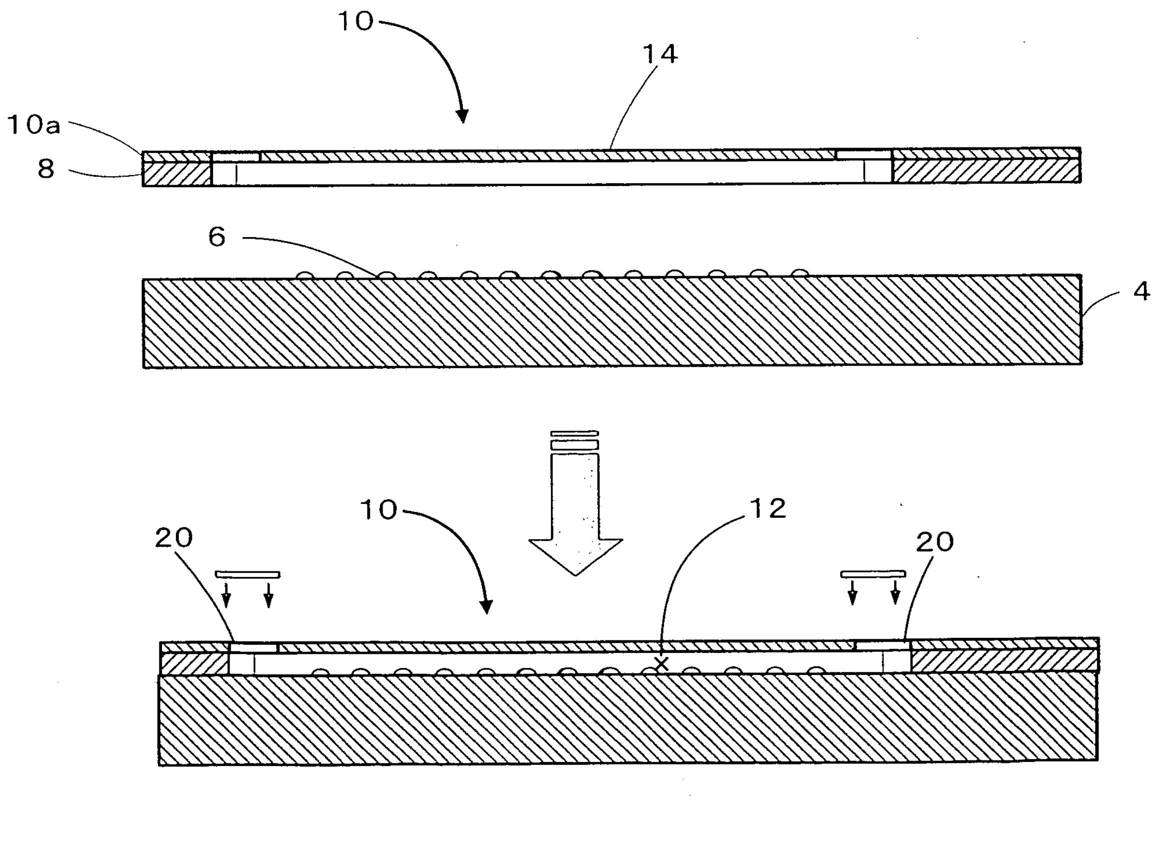

FIG. 5

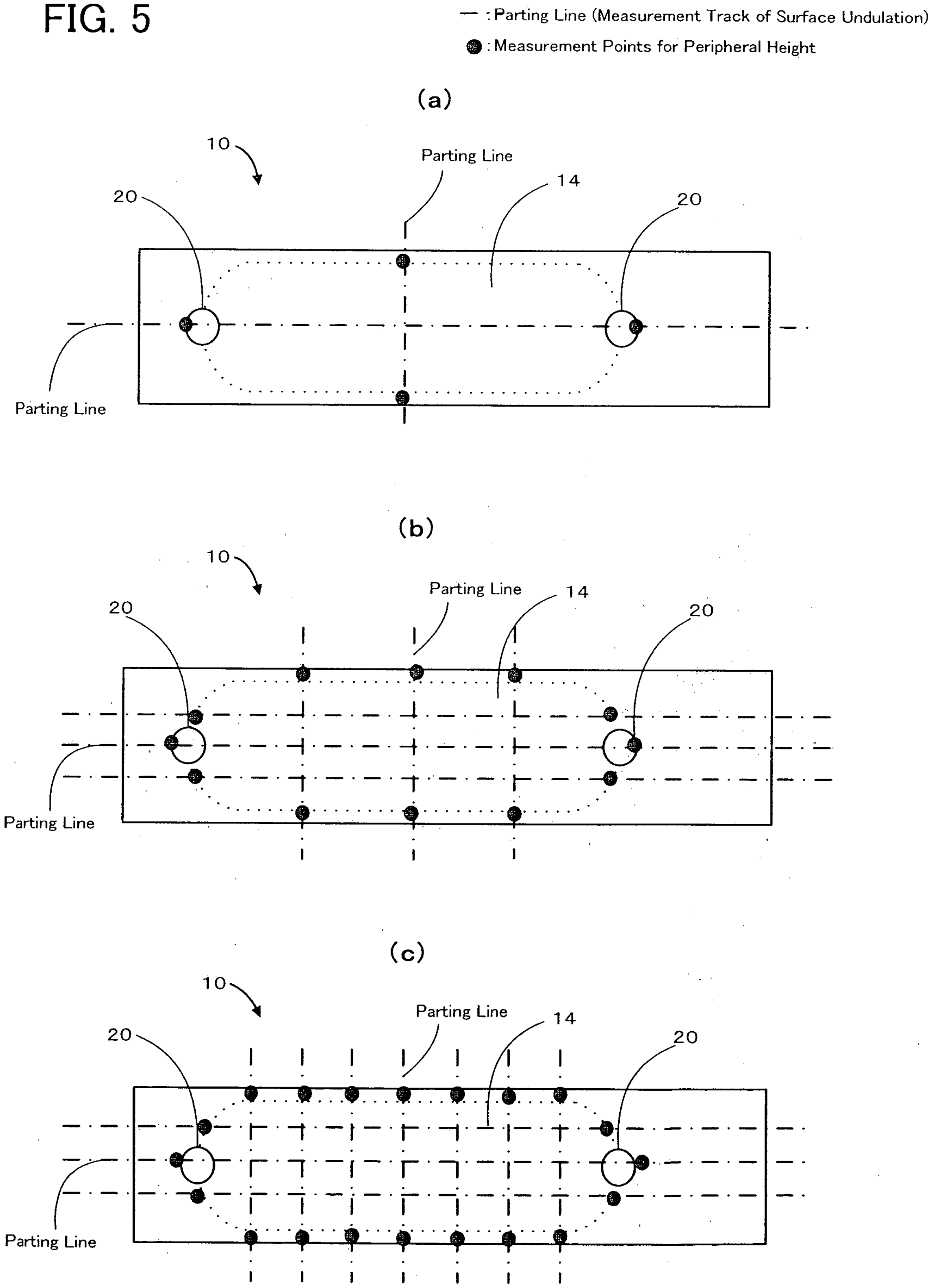
— : Parting Line (Measurement Track of Surface Undulation)
● : Measurement Points for Peripheral Height
(a)
10
Parting Line
14
20
20
Parting Line
(b)
10
Parting Line
14
20
20
Parting Line
(c)
10
Parting Line
14
20
20
Parting Line

FIG. 6

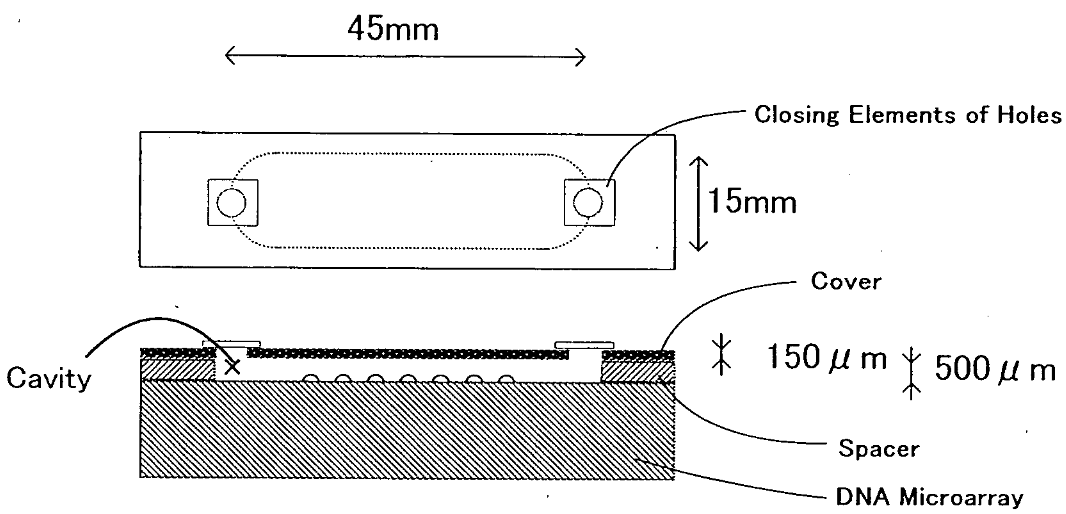
45mm
Closing Elements of Holes
15mm
Cover
Cavity
150 μm
500 μm
Spacer
DNA Microarray

# FIG. 7

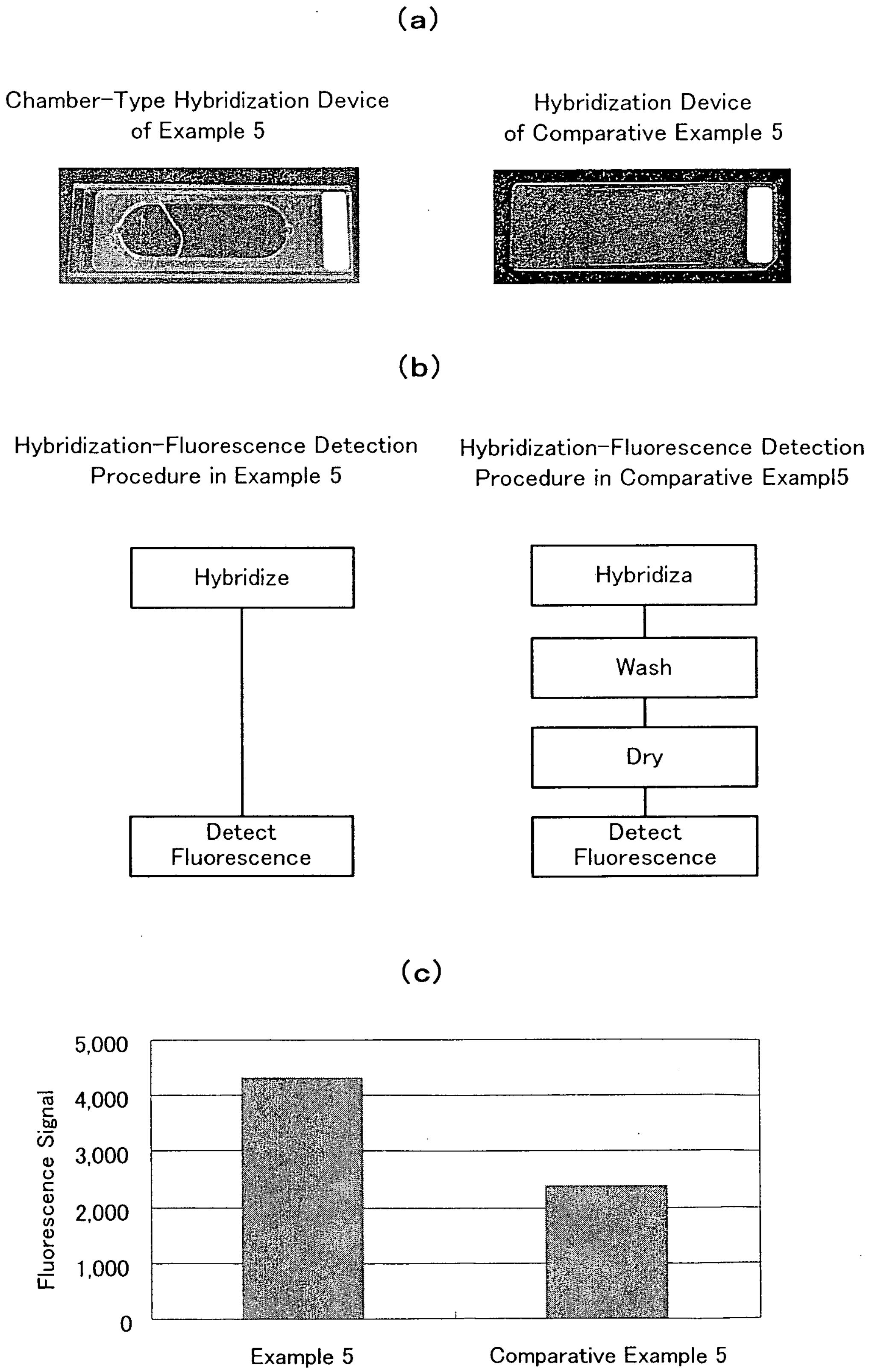
(a)
Chamber-Type Hybridization Device of Example 5
Hybridization Device of Comparative Example 5
(b)
Hybridization-Fluorescence Detection Procedure in Example 5
Hybridization-Fluorescence Detection Procedure in Comparative Exampl5
Hybridize
Detect Fluorescence
Hybridiza
Wash
Dry
Detect Fluorescence
(c)
Fluorescence Signal
5,000
4,000
3,000
2,000
1,000
0
Example 5
Comparative Example 5

FIG. 8

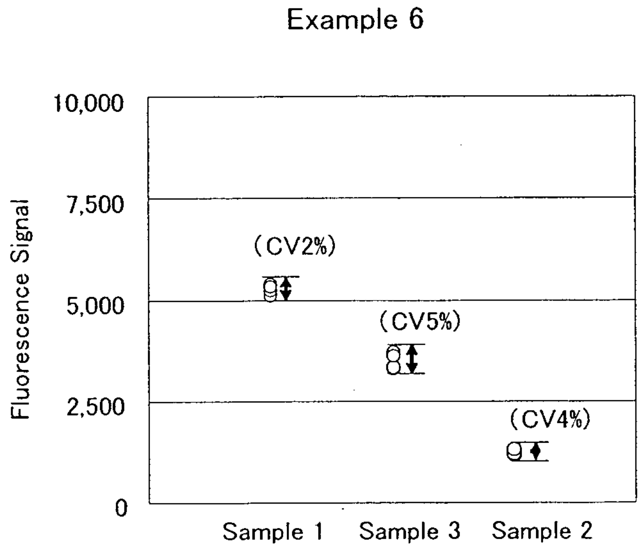
Example 6
10,000
7,500
5,000
2,500
0
Fluorescence Signal
(CV2%)
(CV5%)
(CV4%)
Sample 1
Sample 3
Sample 2

FIG. 9

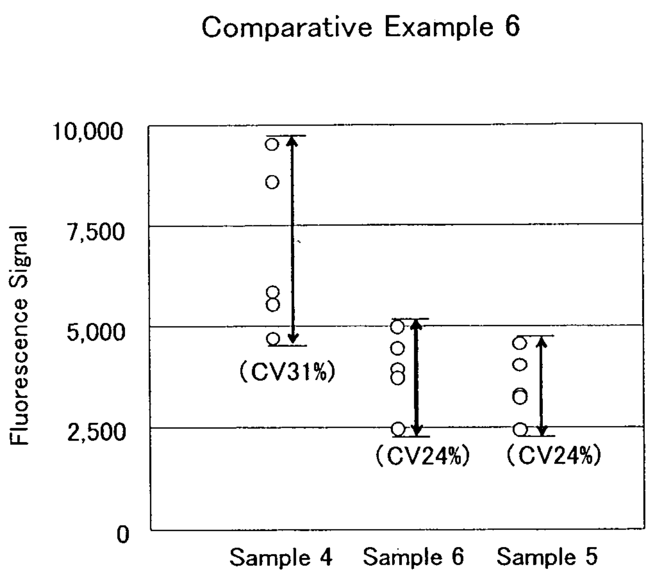
Comparative Example 6
10,000
7,500
5,000
2,500
0
Fluorescence Signal
(CV31%)
(CV24%)
(CV24%)
Sample 4
Sample 6
Sample 5

FIG. 10

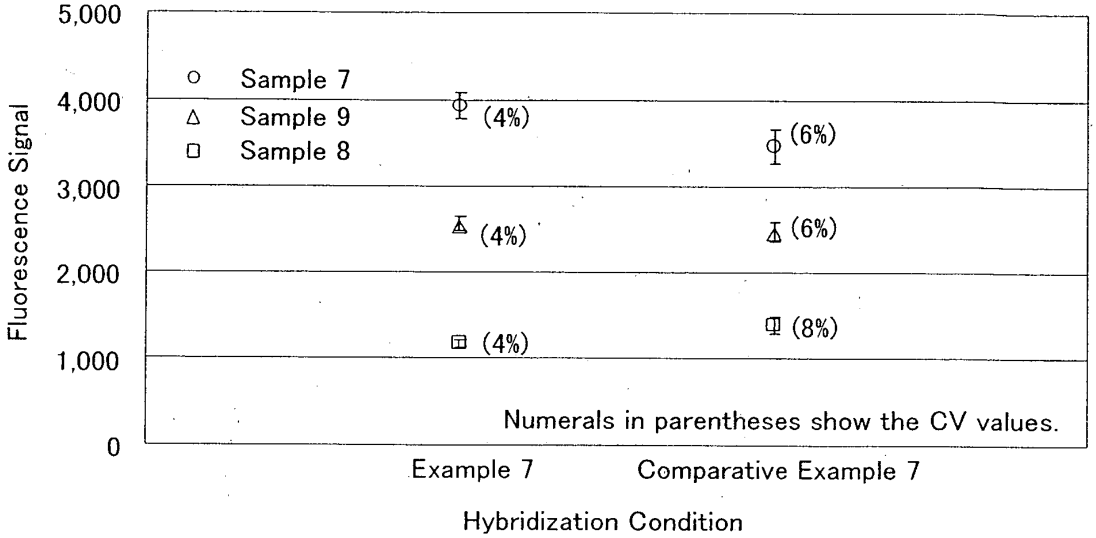
5,000
4,000
3,000
2,000
1,000
0
Fluorescence Signal
Sample 7
Sample 9
Sample 8
(4%)
(6%)
(4%)
(6%)
(4%)
(8%)
Numerals in parentheses show the CV values.
Example 7
Comparative Example 7
Hybridization Condition

FIG. 11

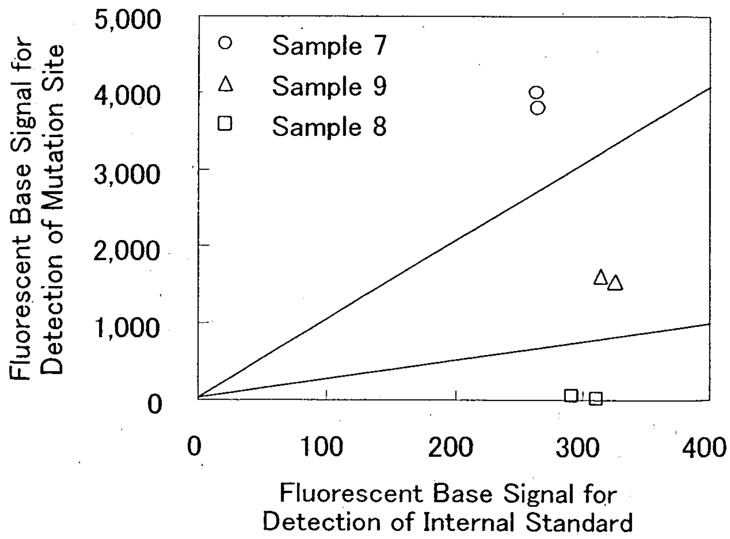
5,000
4,000
3,000
2,000
1,000
0
Fluorescent Base Signal for Detection of Mutation Site
Sample 7
Sample 9
Sample 8
0
100
200
300
400
Fluorescent Base Signal for Detection of Internal Standard

FIG. 12

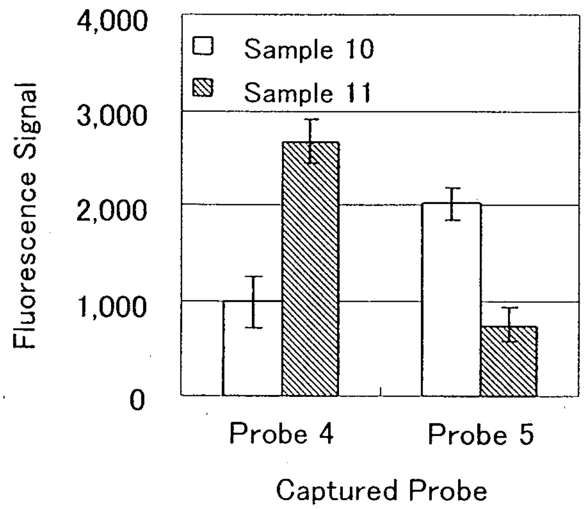
4,000
3,000
2,000
1,000
0
Fluorescence Signal
Sample 10
Sample 11
Probe 4
Probe 5
Captured Probe

FIG. 13

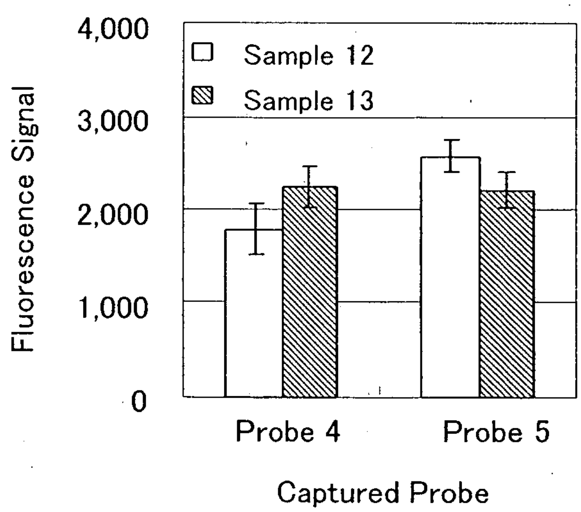
4,000
3,000
2,000
1,000
0
Fluorescence Signal
Sample 12
Sample 13
Probe 4
Probe 5
Captured Probe

FIG. 14

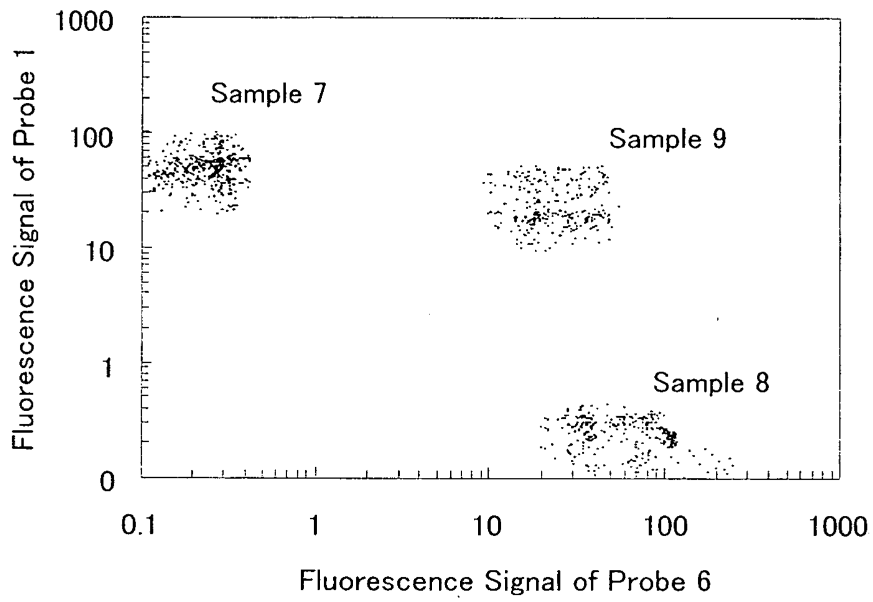
1000
100
10
1
0
Fluorescence Signal of Probe 1
Sample 7
Sample 9
Sample 8
0.1
1
10
100
1000
Fluorescence Signal of Probe 6

FIG. 15

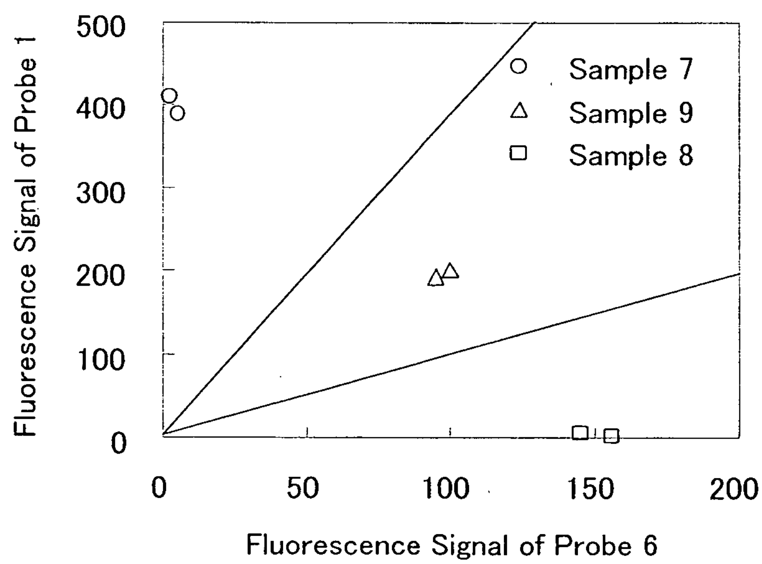
500
400
300
200
100
0
Fluorescence Signal of Probe 1
Sample 7
Sample 9
Sample 8
0
50
100
150
200
Fluorescence Signal of Probe 6

**INTERNATIONAL SEARCH REPORT**

International application No.
PCT/JP2006/300431

A. CLASSIFICATION OF SUBJECT MATTER
***C12N15/09***(2006.01), ***C12M1/00***(2006.01), ***C12Q1/68***(2006.01), ***G01N33/53***(2006.01), ***G01N37/00***(2006.01)

According to International Patent Classification (IPC) or to both national classification and IPC

B. FIELDS SEARCHED

Minimum documentation searched (classification system followed by classification symbols)
***C12N15/09***(2006.01), ***C12M1/00***(2006.01), ***C12Q1/68***(2006.01), ***G01N33/53***(2006.01), ***G01N37/00***(2006.01)

Documentation searched other than minimum documentation to the extent that such documents are included in the fields searched

Electronic data base consulted during the international search (name of data base and, where practicable, search terms used)
BIOSIS/WPI(DIALOG)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2004/058793 A1 (NGK Insulators, Ltd.), 15 July, 2004 (15.07.04), & US 2004/0186281 A1 & US 2005/0059037 A1 | 28-49,66-67<br>1-27,50-65 |
| Y | JP 2003-021637 A (Fuji Photo Film Co., Ltd.), 24 January, 2003 (24.01.03), (Family: none) | 1-27,50-65 |
| Y | JP 2004-177345 A (Sumitomo Bakelite Co., Ltd.), 24 June, 2004 (24.06.04), (Family: none) | 1-27,50-65 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 March, 2006 (10.03.06) | 20 March, 2006 (20.03.06) |
| Name and mailing address of the ISA/<br>Japanese Patent Office | Authorized officer |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2004357570 A
- JP 2004168672 A
- JP 2004166522 A
- JP 2002281978 A
- JP 2005004703 A
- JP 2004016602 W
- WO 2004058793 A
- US 60435995 B
- JP 2003314556 A
- US 60523318 B
- JP 2003016602 W
- US 10795436 B
- JP 2005004703 W
- US 60643603 B
- US 60660224 B